# EUROPÄISCHE PATENTSCHRIFT

(11) **EP 1 368 402 B1**
(45) Veröffentlichungstag und Bekanntmachung des Hinweises auf die Patenterteilung: **11.08.2004**
(21) Anmeldenummer: 02703598.9
(22) Anmeldetag: 13.02.2002
(51) Int. Cl.: C08G 59/30, C08G 59/32, C08L 63/00, A61K 6/087

(54) **POLYMERISIEBARE ZUBEREITUNGEN AUF DER BASIS VON SILIZIUMVERBINDUNGEN MIT ALIPHATISCHEN UND CYCLOALIPHATISCHEN EPOXIDGRUPPEN**
POLYMERIZABLE PREPARATIONS ON THE BASIS OF SILICON COMPOUNDS COMPRISING ALIPHATIC AND CYCLOALIPHATIC EPOXIDE GROUPS
PREPARATIONS POLYMERISABLES SE BASANT SUR DES COMPOSES DE SILICIUM COMPRENANT DES GROUPES EPOXYDE ALIPHATIQUES ET CYCLOALIPHATIQUES

(30) Priorität: 19.02.2001 DE 10107985
(43) Veröffentlichungstag der Anmeldung: 10.12.2003
(73) Patentinhaber: 3M ESPE AG, 82229 Seefeld (DE)
(72) Erfinder: KLETTKE, Thomas, 86938 Schondorf (DE); DEDE, Karsten, 86899 Landsberg (DE)
(86) Internationale Anmeldenummer: PCT/EP2002/001491
(87) Internationale Veröffentlichungsnummer: WO 2002/066535

(56) Entgegenhaltungen:
- EP-A- 0 897 710
- WO-A-00/19967
- WO-A-98/33645
- DE-A- 19 648 283
- US-A- 5 639 413

## Beschreibung

Die Erfindung betrifft polymerisierbare Zubereitungen auf der Basis von siliziumhaltigen Epoxiden und ihre Verwendung.

In polymerisierbaren Dentalmassen wurden bislang vorwiegend Methacrylat- und Acrylatmonomere verwendet. Besondere Aufmerksamkeit verdient das von Bowen beschriebene 2,2-Bis[4,1 -phenylenoxy(2-hydroxy-3,1 -propandiyl)-methacrylsäureester]-propyliden (Bis-GMA) (US-A-3 066 112). Mischungen dieses Methacrylats mit Triethylenglykoldimethacrylat dienen auch heute noch als Monomermatrix für dentale plastische Direkt-Füllungswerkstoffe. Auch Methacrylderivate des zweifach formylierten Bis-(hydroxymethyl)tricyclo[5.2.1.0^{2,6}]-decans haben sich als Monomere für Dentalcomposite bewährt (W. Gruber et al., DE-A-27 14 538; W. Schmitt et al., DE-C-28 16 823; J. Reiners et al., EP-A-0 261 520). Ein großer Nachteil der bekannten polymerisierbaren Dentalmassen ist der Polymerisationsschrumpf, der beispielsweise bei der Anwendung als Füllungsmaterial durch die Bildung von Randspalten Sekundärkaries verursachen kann. Weiterhin führt bei Dentalmassen auf Acrylatbasis die Polymerisationsinhibierung durch Sauerstoff zur Ausbildung einer sogenannten Schmierschicht, die beispielsweise bei Füllungen unerwünscht bzw. sogar schädlich ist.

Obwohl es umfangreiche Erfahrungen mit Epoxiden und cycloaliphatischen Epoxiden gibt (US-A-2 716 123, US-A-2 750 395, US-A-2 863 881, US-A-3 187 018), sind solche Monomere und daraus formulierte kationisch polymerisierbare Massen mit den für dentale Anwendungen notwendigen Eigenschaften zu keinem Zeitpunkt kommerziell verfügbar gewesen.

Die Herstellung bifunktioneller cycloaliphatischer Epoxide ist bereits seit geraumer Zeit bekannt (US-A-2 750 395, US-A-900 506, US-A-907 149, US-A-2 745 847, US-A- 2 853, 499, US-A-3 187 018, US-A-2 863 881, US-A-2 853 498). Siliziumhaltige cycloaliphatische Epoxide zur Herstellung dreidimensionaler Objekte mittels Stereolithographie wurden von Crivello et al. in verschiedenen Publikationen beschrieben (WO 96/30182, EP-A-0 449 027; J.Polym.Sci., Part A: Polym.Chem. 28 (1990) 479, ibid. 31(1993)2563; ibid. 31 (1993) 2729; ibid. 31 (1993) 3109; ibid. 31 (1993) 3121; ibid. 33 (1995) 2463). Siliziumhaltige Epoxide, die Aromaten enthalten, sind als Monomere zur Herstellung schwingungsdämpfender Oberflächen beschrieben worden (US-A-4 902 3689).

EP 412 420 A1, WO 95/25139 und GB 1 123 960 beschreiben ebenfalls siliziumhaltige Epoxide.

Ein Verfahren zur Herstellung von Silikonmaterialien, die eine aliphatische und eine cycloaliphatische Epoxyfunktion enthalten, wurde von Crivello et al. beschrieben (J. Polym. Sci.: Part A, 1993, 3109-31119 sowie US-A 5 484 950).

Kationisch härtbare Epoxidmassen für dentale Anwendungen sind z.B. aus der US-A-5 556 896 bekannt. Diese Druckschrift beschreibt epoxidhaltige Massen, die notwendigerweise als schrumpfkompensierende Monomere Spiroorthocarbonate enthalten müssen.

Desweiteren werden in der WO 95/30402 photopolymerisierbare Verbindungen beschrieben, die Epoxidmonomere enthalten. Die dort beschriebenen Massen sind aufgrund ihrer hohen Wasseraufnahme im polymerisierten Zustand für dentale Anwendungen im Mundmilieu ungeeignet.

Die in der DE-A-4 340 949 beschriebenen cycloaliphatischen Epoxiden sind im wesentlichen niedermolekulare Monomere, die zwar einen verminderten Polymerisationsschrumpf besitzen aber aufgrund ihrer toxikologischen Eigenschaften den Anforderungen an Werkstoffe für dentale Anwendungen nicht genügen.

In den Schriften WO 98/47046, WO 98/47047 und EP-A-0 897 710 werden Epoxidmassen für dentale Anwendungen beschrieben, die sich durch ein neues Initiatorsystem auszeichnen, aber auf herkömmlichen Epoxidmonomeren basieren.

Die WO 98/22521 beschreibt polymerisierbare Massen auf der Basis von Epoxiden, unter anderem auch für die dentale Anwendung. Nachteilig an den dort offenbarten Epoxidmassen sind die relativ hohe Viskosität und die moderate Reaktivität der monomerhaltigen Massen.

Dem Fachmann ist außerdem bekannt, dass cycloaliphatische Epoxidmonomere wie Cyclohexenoxidderivate, gegenüber den aliphatischen Epoxidmonomeren aufgrund der höheren Ringspannung eine gesteigerte Reaktivität aufweisen. Cycloaliphatische Derivate werden daher für die Verarbeitung zu polymerisierbaren Massen bevorzugt, wenn ein hoher Polymerisationsgrad erreicht werden soll.

Die Aufgabe der vorliegenden Erfindung ist es, Zubereitungen bereitzustellen, die sich durch gute Handlingseigenschaften, gute Verarbeitbarkeit, durch geringen Volumenschrumpf und hohe Reaktivität bei der Polymerisation sowie im polymerisierten Zustand durch eine hohe Stabilität im Mundmilieu auszeichnen. Dazu sollen die Monomere eine geringe Viskosität bei gleichzeitig guten mechanischen Werten der diese Monomere enthaltenden Zubereitungen aufweisen. Weiterhin sollen die Zubereitungen eine für dentale Anwendungen ausreichende Lagerstabilität aufweisen.

Erfindungsgemäß wird diese Aufgabe gelöst durch polymerisierbare Zubereitungen, enthaltend
(a) 1 bis 80 Gew.-% eines Epoxids oder eines Gemisches von Epoxiden der allgemeinen Formel: 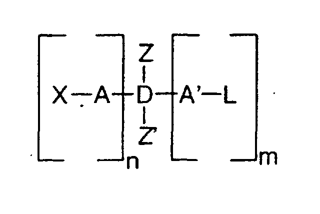 wobei
   A, A' unabhängig voneinander einen unverzweigten oder verzweigten aliphatischen, cycloaliphatischen oder aromatischen Rest oder eine Kombination dieser Reste mit 0 bis 16 C-Atomen bedeuten, wobei ein oder mehrere C-Atome durch O, O(C=O), C=O, Si, N, S ersetzt sein können,
   X einen unverzweigten oder verzweigten aliphatischen oder aromatischen Rest oder eine Kombination dieser Reste mit 3 bis 16 C-Atomen bedeutet, wobei ein oder mehrere C-Atome durch O, O(C=O), (C=O), Si, N, S ersetzt sein können und eine aliphatische end- oder mittelständige Epoxid-Gruppe enthalten ist,
   L einem upverzweigten oder verzweigten aliphatischen cycloaliphatischen oder aromatischen Rest oder eine Kombination dieser Reste mit 6 bis 30 C-Atomen bedeutet, wobei ein oder mehrere C-Atome durch O, O(C=O), (C=O), Si, N, S ersetzt sein können und eine cycloaliphatische Epoxidgruppe enthalten ist,
   Z, Z' unabhängig voneinander H oder einen unverzweigten oder verzweigten aliphatischen, cycloaliphatischen oder aromatischen Rest oder eine Kombination dieser Reste mit 0 bis 10 C-Atomen bedeuten, wobei ein oder mehrere C-Atome durch O, O(C=O), (C=O), Si, N, S ersetzt sein können,
   D einen unverzweigten oder verzweigten aliphatischen, cycloaliphatischen oder aromatischen Rest oder eine Kombination dieser Reste mit 1 bis 55 C-Atomen bedeutet, wobei mindestens ein C-Atom durch SiGG', SiG oder Si ersetzt ist und ein oder mehrere C-Atome durch O, O(C=O), (C=O), N oder S ersetzt sein können,
   G, G' unabhängig voneinander, einen unverzweigten oder verzweigten aliphatischen, cycloaliphatischen oder aromatischen Rest oder eine Kombination dieser Reste mit 1 bis 15 C-Atomen bedeuten, wobei ein oder mehrere C-Atome durch O, O(C=O), (C=O), Si, N, S ersetzt sein können,
   n und m unabhängig voneinander 1, 2, 3 oder 4 bedeuten
   und wobei die Molmasse des Epoxids oder die mittlere Molmasse des Gemisches von Epoxiden 360 bis 3000 g/mol beträgt,
(b) 0 bis 80 Gew.-% eines Epoxids oder eines Gemisches von Epoxiden, die von (a) verschieden sind,
(c) 3 bis 85 Gew.-% Füllstoffe,
(d) 0,01 bis 25 Gew.-% Initiatoren, Verzögerer und/oder Beschleuniger,
(e) 0 bis 25 Gew.-% Hilfsstoffe,
wobei die Prozentangaben jeweils auf das Gesamtgewicht der Zubereitung bezogen sind.

Überraschenderweise zeigen die erfindungsgemäßen Zubereitungen mit der Kombination von aliphatischen und cycloaliphatischen Epoxidgruppen mit einer mittleren Molmasse der polymerisierbaren Verbindung von 200 bis 3000 g/mol auch bei einer für die Verarbeitung angenehmen Konsistenz eine sehr geringe Klebrigkeit bei guten mechanischen Eigenschaften der polymerisierten Zubereitung.

Bevorzugte polymerisierbare Zubereitungen enthalten
(a) 3 bis 50 Gew.-% eines Epoxids oder eines Gemisches von Epoxiden der allgemeinen Formel: 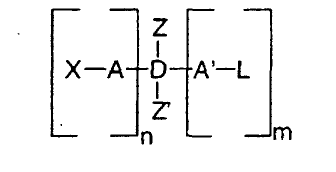 wobei
   A, A' unabhängig voneinander einen unverzweigten oder verzweigten aliphatischen, cycloaliphatischen oder aromatischen Rest oder eine Kombination dieser Reste mit 0 bis 12 C-Atomen bedeuten, wobei ein oder mehrere C-Atome durch O, O(C=O), C=O, Si, N, S ersetzt sein können,
   X einen unverzweigten oder verzweigten aliphatischen oder aromatischen Rest oder eine Kombination dieser Reste mit 3 bis 14 C-Atomen bedeuten, wobei ein oder mehrere C-Atome durch O, O(C=O), (C=O), Si, N, S ersetzt sein können und eine aliphatische end- oder mittelständige Epoxid-Gruppe enthalten ist,
   L einen unverzweigten oder verzweigten aliphatischen, cycloaliphatischen oder aromatischen Rest oder eine Kombination dieser Reste mit 6 bis 20 C-Atomen bedeutet, wobei ein oder mehrere C-Atome durch O, O(C=O), (C=O), Si, N, S ersetzt sein können und eine cycloaliphatische Epoxidgruppe enthalten ist,
   Z, Z' unabhängig voneinander H oder einen unverzweigten oder verzweigten aliphatischen, cycloaliphatischen oder aromatischen Rest oder eine Kombination dieser Reste mit 0 bis 10 C-Atomen bedeuten, wobei ein oder mehrere C-Atome durch O, O(C=O), (C=O), Si ersetzt sein können,
   D einen unverzweigten oder verzweigten aliphatischen, cycloaliphatischen oder aromatischen Rest oder eine Kombination dieser Reste mit 1 bis 50 C-Atomen bedeutet, wobei mindestens ein C-Atom durch SiGG', SiG oder Si ersetzt ist und ein oder mehrere C-Atome durch O, O(C=O), (C=O) ersetzt sein können,
   G, G' unabhängig voneinander einen unverzweigten oder verzweigten aliphatischen, cycloaliphatischen oder aromatischen Rest oder eine Kombination dieser Reste mit 1 bis 8 C-Atomen bedeuten, wobei ein oder mehrere C-Atome durch O, O(C=O), (C=O), Si ersetzt sein können,
   n und m unabhängig voneinander 1, 2, 3 oder 4 bedeuten,
   wobei die Molmasse des Epoxids oder die mittlere Molmasse des Gemisches von Epoxiden 360 bis 2500 g/mol beträgt,
(b) 0 bis 60 Gew.-% eines Epoxids oder eines Gemisches von Epoxiden, die von (a) verschieden sind,
(c) 15 bis 85 Gew.-% Füllstoffe,
(d) 0,01 bis 20 Gew.-% Initiatoren, Verzögerer und/oder Beschleuniger,
(e) 0 bis 25 Gew.-% Hilfsstoffe,
   wobei die Prozentangaben jeweils auf das Gesamtgewicht der Zubereitung bezogen sind.

Insbesondere bevorzugt sind Zubereitungen, deren Komponente (a) eine Molmasse des Epoxids oder eine mittlere Molmasse des Gemisches von Epoxiden von 360 bis 2500 g/mol aufweist.

Dabei ist bei den erfindungsgemäßen Zubereitungen mit der Kombination von aliphatischen und cycloaliphatischen Epoxidgruppen mit einer mittleren Molmasse der polymerisierbaren Verbindung von 360 bis 2500 g/mol zusätzlich ein höherer Füllgrad bei angenehmer Verarbeitbarkeit und Konsistenz unter Beibehaltung der geringen Klebrigkeit erreichbar.

Geeignete Zubereitungen können insbesondere als Komponente (a) eines oder mehrere Epoxide nachstehenden Formel enthalten 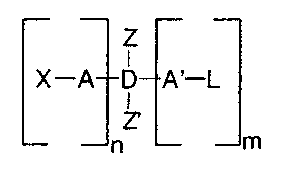 wobei
A, A' unabhängig voneinander einen unverzweigten oder verzweigten aliphatischen, cycloaliphatischen oder aromatischen Rest oder eine Kombination dieser Reste mit 0 bis 12 C-Atomen bedeuten, wobei ein oder mehrere C-Atome durch O, O(C=O), C=O, Si, N, S ersetzt sein können,
X einen unverzweigten oder verzweigten aliphatischen Rest oder eine Kombination dieser Reste mit 3 bis 14 C-Atomen bedeutet, wobei ein oder mehrere C-Atome durch O, O(C=O), (C=O), Si, N, S ersetzt sein können und mindestens eine endständige aliphatische Epoxidgruppe enthalten ist,
L einen unverzweigten oder verzweigten aliphatischen, cycloaliphatischen Rest oder eine Kombination dieser Reste mit 7 bis 15 C-Atomen bedeutet, in dem ein oder mehrere C-Atome durch O, O(C=O), (C=O), Si, N, S ersetzt sein können, und eine cycloaliphatische Epoxidgruppe der nachfolgenden Formel enthalten ist. 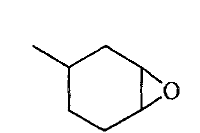 D einen unverzweigten oder verzweigten aliphatischen, cycloaliphatischen oder aromatischen Rest oder eine Kombination dieser Reste mit 1 bis 50 C-Atomen bedeutet, wobei mindestens ein C-Atom durch SiGG', SiG oder Si ersetzt ist und ein oder mehrere C-Atome durch O, O(C=O), (C=O) ersetzt sein können,
G, G' unabhängig voneinander einen unverzweigten oder verzweigten aliphatischen oder aromatischen Rest oder eine Kombination dieser Reste mit 1 bis 8 C-Atomen bedeuten, wobei ein oder mehrere C-Atome durch O, O(C=O), (C=O), Si ersetzt sein können,
n und m unabhängig voneinander 1, 2, 3 oder 4 bedeuten,
wobei die Molmasse des Epoxids oder die mittlere Molmasse des Gemisches von Epoxiden 360 bis 2500 g/mol beträgt.

Überraschend wurde festgestellt, dass die erfindungsgemäße polymerisierbare Zubereitung dann besonders gute Handlingseigenschaften aufweist, wenn sie als Komponente (a) eines oder mehrere Epoxide entsprechend der nachstehenden Formel enthält: 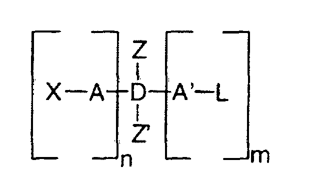 wobei
A, A' unabhängig voneinander einen unverzweigten oder verzweigten aliphatischen, cycloaliphatischen oder aromatischen oder eine Kombination dieser Reste Rest mit 0 bis 10 C-Atomen bedeuten, wobei ein oder mehrere C-Atome durch O, O(C=O), C=O, Si ersetzt sein können,
X einen verzweigten oder unverzweigten aliphatischen Rest mit 3 bis 14 Kohlenstoffatomen bedeutet, in dem ein oder mehrere Kohlenstoffatome durch O, O(C=O), (C=O), Si ersetzt sein können und mindestens eine endständige aliphatische Epoxidgruppe enthalten ist,
L einen unverzweigten oder verzweigten aliphatischen oder cycloaliphatischen Rest oder eine Kombination dieser Reste mit 7 bis 15 C-Atomen bedeutet, in dem ein oder mehrere C-Atome durch O, O(C=O), (C=O), Si ersetzt sein können, und eine cycloaliphatische Epoxidgruppe der nachfolgenden Formel enthalten ist, 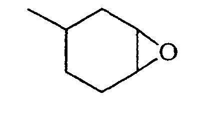 D einen unverzweigten oder verzweigten aliphatischen, cycloaliphatischen oder aromatischen Rest oder eine Kombination dieser Reste mit 1 bis 50 C-Atomen bedeutet, wobei mindestens ein C-Atom durch SiGG', SiG oder Si ersetzt ist und ein oder mehrere C-Atome durch O, O(C=O), (C=O) ersetzt sein können,
G, G' unabhängig voneinander einen unverzweigten oder verzweigten aliphatischen oder aromatischen Rest oder eine Kombination dieser Reste mit 1 bis 8 C-Atomen bedeuten,
n und m unabhängig voneinander 1, 2, 3 oder 4 bedeuten und m+n 2 bis 6 ergeben,
und wobei die Molmasse des Epoxids oder die mittlere Molmasse des Gemisches von Epoxiden 360 bis 2500 g/mol beträgt.

Besonders bevorzugt bei den Epoxiden der Komponente (a) sind solche, die einen der folgenden Bestandteile D aufweisen, der jeweils über das Si-Atom an die Bestandteile A, A' angebunden ist:

i. Si

ii. SiG

iii. SiGG'

wobei G, G' unabhängig voneinander einen unverzweigten oder verzweigten aliphatischen, cycloaliphatischen oder aromatischen Rest mit 1 bis 8 C-Atomen oder eine Kombination dieser Reste bedeuten, wobei ein oder mehrere C-Atome durch O, O(C=O), (C=O), Si ersetzt sein können.

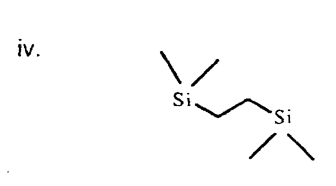

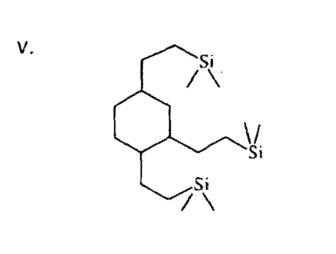

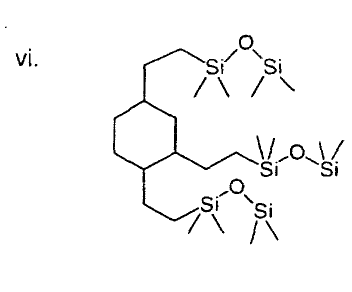

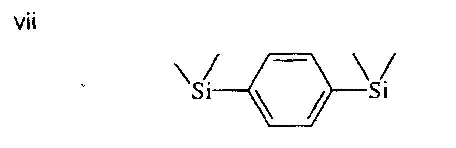

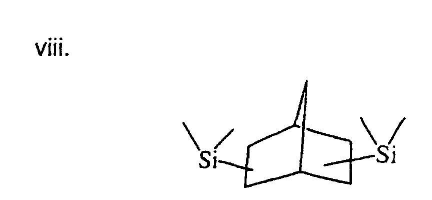

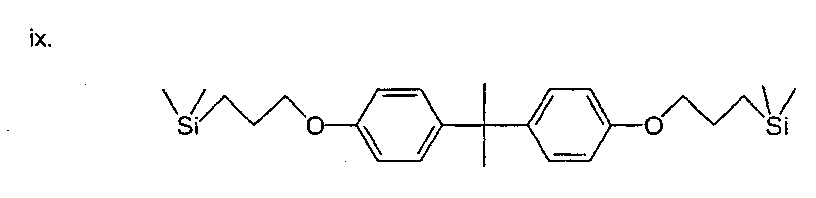

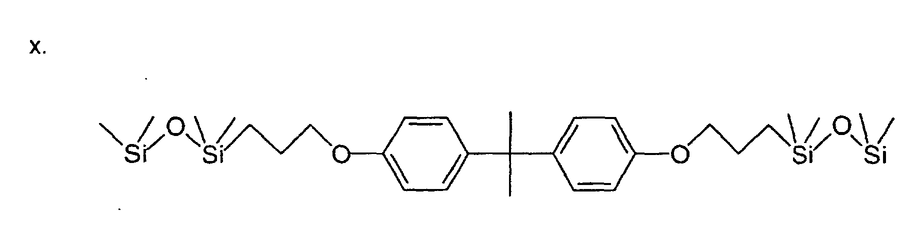

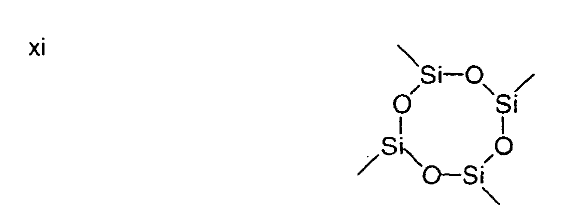

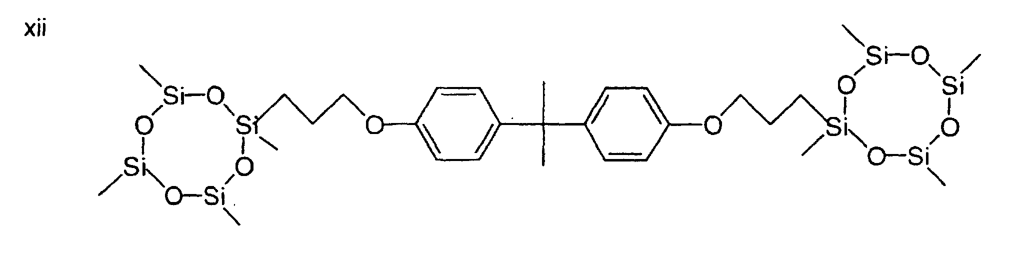

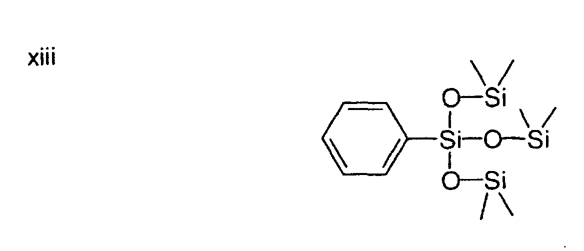

Von den Komponenten (a) sind solche bevorzugt, die Z= 0 aufweisen und in denen G bzw. G' eine Methylgruppe darstellt.

Zu besonders guten Ergebnissen führen beispielsweise aber ohne Einschränkung Zubereitungen, die eines oder mehrere der folgenden Epoxide enthalten:
i. Silane, 1,2-ethylene-[1,2-dimethyl-1-[3-(oxiranylmethoxy)propyl]-2-[2-(7-oxabicyclo[4.1.0]hept-3-yl)ethyl)- 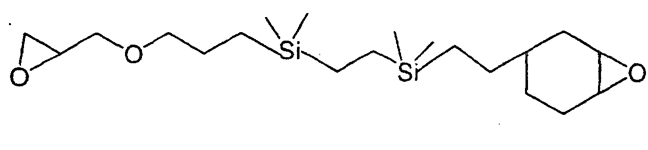
ii. Silane, 1,1',1"-(1,2,4-cyclohexylene-1-(dimethyl[3-(oxiranylmethoxy)propyl])-2,4-bis[2-(7-oxabicyclo[4.1 .0]hept-3-yl)ethyl])- 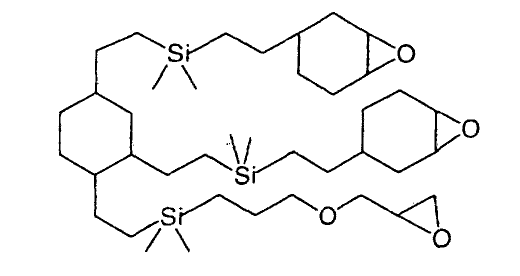
iii. Disiloxane, 1,1',1 "-(1,2,4-cyclohexanetriyltri-2,1-ethanediyl)-1,1,3,3-tetramethyl-3-(oxiranylmethoxy)propylbis[1,1,3,3-tetramethyl-2-(7-oxabicyclo[4.1.0]hept-3-yl)ethyl]- 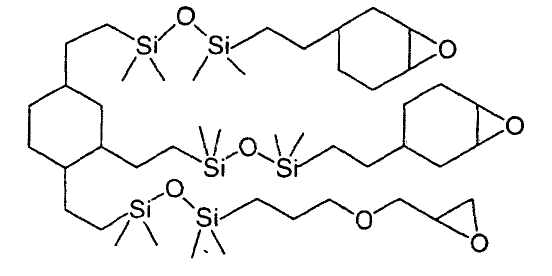
iv. Silane, 1,4-phenylenebis[dimethyl-1-[3-(oxiranylmethoxy)propyl-4- [2-(7-oxabicyclo[4.1.0]hept-3-yl)ethyl]- 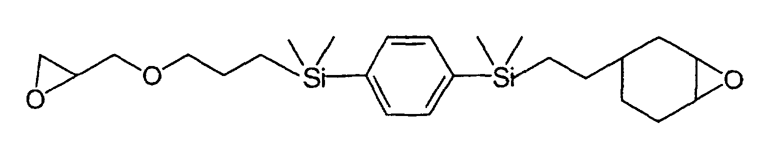
v. Silane, 2,5-bicyclo[2.2.1.]heptylenebis[dimethyl-2-[3-(oxiranylmethoxy)propyl]-5-[2-(7-oxabicyclo[4.1.0]hept-3-yl)ethyl]- 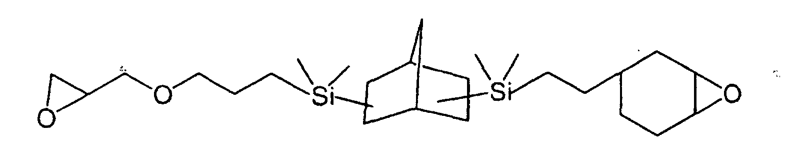
vi. Silane, 1,1'-[bis(4,4'-hydroxyphenyl-2,2-propane-3,1-propanediyl)]-1-dimetyl[3-(oxiranylmethoxy)propyl]-1'-dimethyl[2-(7-oxabicyclo[4.1.0]hept-3-yl)ethyl]- 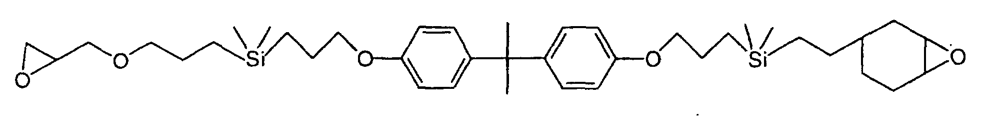
vii. Siloxane, 1,1'-[bis(4,4'-hydroxyphenyl-2,2-propane-3,1-propanediyl)-1,1,3,3-tetrametyl]-1-[3-(oxiranylbutyl]-1 '-[2-(7-oxabicyclo[4.1.0]hept-3-yl)ethyl]- 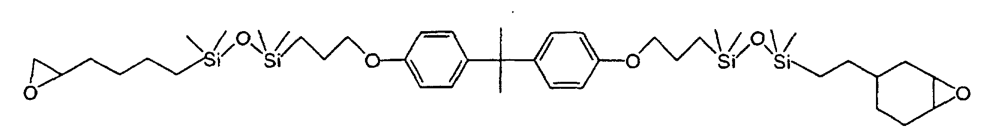
viii. Cyclotetrasiloxane, 2,4,6,8-tetramethyl-2,4,6-tris[2-(7-oxabicyclo[4.1.0]hept-3-yl)ethyl]-8-[3-(oxiranylmethoxy)propyl]- 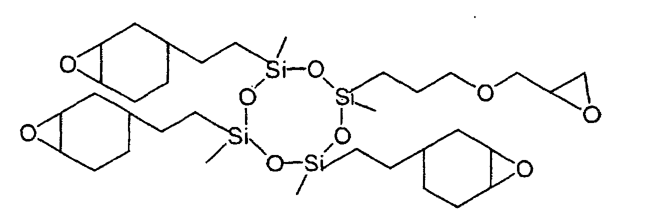
ix. Cyclotetrasiloxane, 2,4,6,8-tetramethyl-2,4,6-tris[2-(7-oxabicyclo[4.1.0]hept-3-yl)ethyl]-8-[4-oxiranylbutyl]- 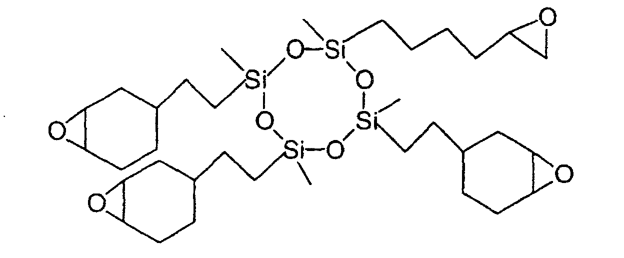
x. Cyclotetrasiloxane, 1,1'-[bis(4,4'-hydroxyphenyl-2,2-propane-3,1-propanediyl)]bis[2,4,6,8-tetramethyl-4,6-bis[3-(oxiranylmethoxy)propyl]-8-[2-(7-oxabicyclo[4.1.0]hept-3-yl)ethyl]- 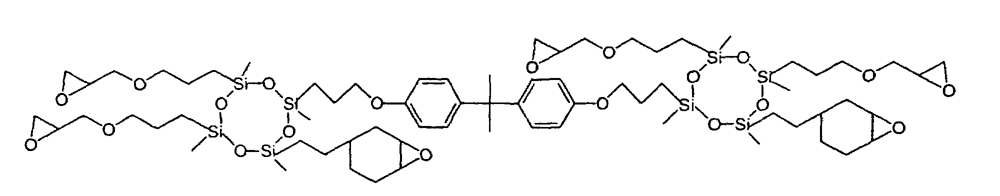
xi. Trisiloxane, 3-[[dimethyl-[2-(7-oxabicyclo[4.1.0]hept-3-yl)ethyl)silyl)oxy]-1,1,5,5-tetramethyl-1-[2-(7-oxabicyclo[4.1.0]hept-3-yl)ethyl]-5-(oxiranylmethoxy)propyl]-3-phenyl- 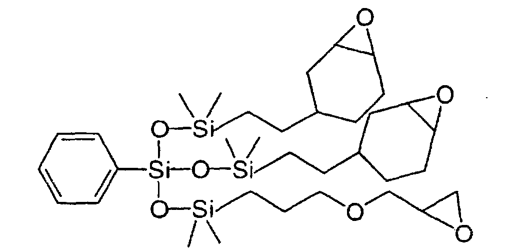
xii. Trisiloxane, 3-[[dimethyl-[2-(7-oxabicyclo[4.1.0]hept-3-yl)ethyl]silyl]oxy]-1,1,5,5-tetramethyl-1-[2-(7-oxabicyclo[4.1.0]hept-3-yl)ethyl]-5-[2-(oxiranylethyl)]-3-phenyl-

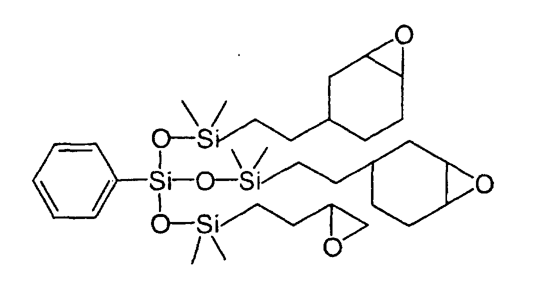

Die erfindungsgemäßen polymerisierbaren Zubereitungen können neben den beschriebenen siliziumhaltigen Epoxiden als Komponente (b) andere Epoxide enthalten. Beispielsweise sind die in US-A-2 716 123, US-A-2 948 688, US-A-2 948 688, US-A-2 985 667, US-A-2 750 395, US-A-2 863 881, US-A-3 187 018 beschriebenen Epoxide als Bestandteile in Komponente (b) der erfindungsgemässen Zubereitung in ihrer gesamten Breite umfaßt. Dazu zählen beispielsweise: 3,4-Epoxycyclohexylmethyl-3,4-epoxycyclohexancarboxylat (US-A-2 716 123), 3,4-Epoxy-6-methylcyclohexyl-3,4-epoxy-6-methylcyclohexancarboxylat (US-A-2 716 123) oder verwandte Epoxide, Vinylcyclohexendiepoxid (US-A-2 948 688), Dicyclopentadiendioxid (US-A-2 985 667), Bis(3,4-epoxycyclohexylmethyl)adipat (US-A-2 750 395, US-A-2 863 881, US-A-3 187 018).

Ebenso umfaßt sind Trisiloxane, 3-[[dimethyl[2-(7-oxabicyclo[4.1.0]hept-3-yl)ethyl]silyl]oxy]-1,1,5,5-tetramethyl-1,5-bis[2-(7-oxabicyclo[4.1.0]hept-3-yl)ethyl]-3-phenyl- (CAS-Nummer 90393-84-3),
7-Oxabicyclo[4.1.0]heptane, 3,3',3",3"'-[(2,4,6,8-tetramethylcyclotetrasiloxan-2,4,6,8-tetrayl)tetra-2,1-ethandiyl]tetrakis- (CAS-Nummer 121225-98-7) der folgenden Formel: 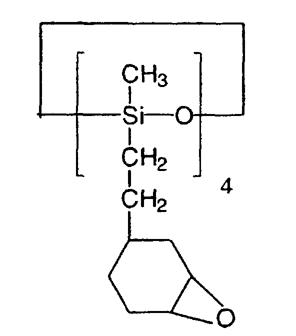 7-Oxabicyclo[4.1.0]heptan, 3,3',3",3"',3""-[(2,4,6,8,10-pentamethylcyclopentasiloxan-2,4,6,8,10-pentayl)penta-2, 1-ethandiyl]pentakis- (CAS-Nummer 141446-51-7) der folgenden Formel:

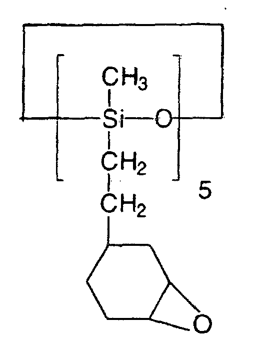

Auch die in WO 98/22521 beschriebenen mehrfunktionellen Epoxide können als Komponente (b) in den polymerisierbaren Massen eingesetzt werden.

Die Epoxide gemäß Komponente (b) können in einer Konzentration von 0 bis 80 Gew.-%, vorzugsweise 0 bis 60 Gew.-%, vorhanden sein, jeweils bezogen auf das Gesamtgewicht der Zubereitung.

Anorganische Füllstoffe gemäß Komponente (c) können übliche dentale Füllstoffe, beispielsweise Quarz, gemahlene, gegebenenfalls röntgenopake, gegebenenfalls reaktive Gläser, schwer lösliche Fluoride, wie CaF₂, YF₃ (EP-B-0 238 025), Kieselgele sowie pyrogene Kieselsäure und/oder deren Granulate sein.

Ebenso können ein oder mehrere wasserlösliche anorganische komplexe Fluoride der allgemeinen Formel AₙMFₘ, worin A ein ein- oder mehrwertiges Kation, M ein Metall der III, IV oder V Haupt-oder Nebengruppe, n eine ganze Zahl von 1 bis 3 und m eine ganze Zahl von 4 bis 6 bedeuten (DE-A-4 445 266), als fluoridabgebende Bestandteile in der Komponente (c) enthalten sein. Sie können in einer Konzentration von 3 bis 85 Gew.-%, vorzugsweise von 5 bis 85 Gew.-% und insbesondere von 30 bis 85 Gew.-%, bezogen auf die Gesamtmasse, in den polymerisierbaren Zubereitungen enthalten sein.

Zum besseren Einbau in die Polymermatrix kann es von Vorteil sein, die genannten Füllstoffe durch übliche Verfahren zu hydrophobieren. Übliche Hydrophobierungsmittel sind Silane, beispielsweise (3-Glycidoxypropyl)trimethoxysilan, 5,6-Epoxyhexyltriethoxysilan oder 2-(3,4-Epoxycyclohexyl)ethyltrimethoxysilan. Die mittlere Korngröße der anorganischen Füllstoffe beträgt vorzugsweise <20 µm, insbesondere <12 µm. Ganz besonders bevorzugt werden Füllstoffe mit einer mittleren Korngröße < 7 µm eingesetzt.

Auch Christobalit, Calciumsilikat, Zirkoniumsilikat, Mondmorillonite wie Bentonite, Zeolithe, einschließlich der Molekularsiebe, wie Natriumaluminiumsilikat, Metalloxidpulver, wie Aluminium- oder Zinkoxide bzw. deren Mischoxide, Bariumsulfat, Calciumcarbonat, Gips und Kunststoffpulver können als Füllstoffe geeignet sein.

Initiatoren gemäß Komponente (d) der erfindungsgemäßen Zubereitungen können sein: Lewis- oder Broensted-Säuren bzw. Verbindungen, die solche Säuren freisetzen, welche die Polymerisation initiieren, beispielsweise BF₃ oder dessen etherische Addukte (BF₃.THF, BF₃.Et₂O, etc.), AlCl₃, FeCl₃, HPF₆, HAsF₆, HSbF₆, HBF₄ oder Substanzen, die nach Bestrahlen durch UV, sichtbares Licht, Wärme und/oder Druck die Polymerisation auslösen, wie z.B. (eta-6-Cumol)(eta-5-cyclopentadienyl)eisenhexafluorophosphat, (eta-6-Cumol)(eta-5-cyclopentadienyl)eisentetrafluoroborat, (eta-6-Cumol)(eta-5-cyclopentadienyl)eisenhexafluoroantimonat, substituierte Diaryliodoniumsalze, Triarylsulfoniumsalze und Pyridiniumsalze. Als Beschleuniger können zum Beispiel Peroxyverbindungen vom Typ der Perester, der Diacylperoxide, der Peroxydicarbonate und der Hydroperoxide eingesetzt werden. Bevorzugt werden Hydroperoxide verwendet und besonders bevorzugt kommt als Beschleuniger Cumolhydroperoxid in etwa 70 bis 90 %iger Lösung in Cumol zum Einsatz. Das Verhältnis von Photoinitiator zu Cumolhydroperoxid kann in weiten Grenzen von 1:0,001 bis 1:10 variiert werden, vorzugsweise wird jedoch ein Verhältnis von 1:0,1 bis 1:6 und besonders bevorzugt von 1:0,5 bis 1:4 verwendet. Die Verwendung von Komplexbildnern, wie beispielsweise Oxalsäure, 8-Hydroxychinolin, Ethylendiamintetraessigsäure und aromatischen Polyhydroxyverbindungen ist ebenfalls möglich.

Ebenso können Initiatorsysteme bestehend aus verschiedenen Komponenten eingesetzt werden, wie sie in EP 0 897 710 A2, WO 98/47046 oder WO 98/47047 beschrieben sind. Bevorzugt werden Initiatorsysteme bestehend aus 1,2-Diketonen (wie Campherchinon), lodoniumiumsalzen mit wenig koordinierenden Anionen (wie Tolylcumyliodoniumietrakis(pentafluorophenyl)borat oder Tolylcumyliodoniumtetrakis(3,5-bis(trifluoromethyl)-phenyl)borate) zusammen mit aromatischen tertiären Aminen (wie 2-Butoxyethyl-4-(dimethylamino)benzoat, Ethyl-4-(dimethylamino)-benzoat) und/oder geeigneten Polyaromaten (wie Anthracen) eingesetzt.

Als Verzögerer können Basen wie beispielsweise tertiäre Amine zugesetzt werden.

Die Komponente (d) liegt in den erfindungsgemäßen Zubereitungen in einer Menge von 0,01 bis 25 Gew.-%, vorzugsweise 0,01 bis 20 Gew.-%, bezogen auf das Gesamtgewicht der Zubereitung, vor.

Geeignete Hilfsstoffe gemäß Komponente (e) können beispielsweise üblicherweise auf dem Dentalgebiet eingesetzte Stabilisatoren (z.B. Tinuvine der Fa. Ciba), Pigmente oder Verdünnungsmittel sein.

Überraschenderweise wurde gefunden, dass die erfindungsgemäßen Zubereitungen mit den siliziumhaltigen Epoxiden gemäß Komponente (a) bei vorteilhaft niedriger Viskosität ebenso gute mechanische Eigenschaften im polymerisierten Zustand aufweisen wie bekannte Dentalmassen.

Weiterhin wurde überraschenderweise gefunden, dass die siliziumhaltigen Epoxide oder Gemische gemäß Komponente (a) der erfindungsgemäßen Zubereitungen trotz hoher Reaktivität eine hervorragende Lagerstabilität besitzen.

Die erfindungsgemäßen epoxidhaltigen, polymerisierbaren Zubereitungen eignen sich insbesondere als Werkstoffe für dentale Zwecke, beispielsweise zur Herstellung von Kunststoffzähnen oder Provisorien, als Beschichtungsmittel, zum Verkleben von Substraten sowie als dentale Füllungsmaterialien. Dabei ist z.B. eine Beschichtung von Kunststoffen, Gläsern, Papier, Folien, Metallen oder mineralischen Substraten möglich. Ferner können beispielsweise Kunststoffe, Gläser, Papier, Folien, Metalle oder mineralische Substrate verklebt werden. Die Verklebung kann dabei kalt, heiß oder durch Bestrahlung oder durch chemische Initiierung erfolgen.

Die polymerisierbare Zubereitung kann als einkomponentiges System zur Verfügung gestellt werden. Ebenso ist eine Formulierung als zwei- oder mehrkomponentiges System denkbar. Dabei können eine oder mehrere Basispasten (A) Epoxide oder Gemische von Epoxiden der Komponenten (a) und (b), einen Teil oder den gesamten Anteil der Füllstoffe der Komponente (c), gegebenenfalls Verzögerer und/oder Beschleuniger gemäß Komponente (d) und gegebenenfalls Hilfsstoffe der Komponente (e) enthalten. Räumlich getrennt hiervon können eine oder mehrere Katalysatorpasten (B) einen oder mehrere Initiatoren gemäß Komponente (d), gegebenenfalls Verzögerer und/oder Beschleuniger gemäß Komponente (d) gegebenenfalls einen Teil der Füllstoffe der Komponente (c) und gegebenenfalls Hilfsstoffe gemäß Komponente (e) aufweisen. Die Pasten (A) und (B) werden dann zum Erhalt der polymerisierbaren Zubereitung miteinander zur Reaktion gebracht. Dies geschieht beispielsweise durch automatisches oder manuelles Mischen von Basis- und Katalysatorpasten.

Die erfindungsgemäße Zubereitung kann in verschiedene Behältnisse abgepackt werden. Geeignet sind z.B. Kartuschen mit einer oder mehreren Kammern, Mischkapseln, Schraubtuben oder Tuben. Femer kann die polymerisierbare Zubereitung in verschiedenen Ausbringvorrichtungen enthalten sein.

In nachstehender Tabelle sind Beispiele für Monomerzusammensetzungen genannt, die die Aufgabe der vorliegenden Erfindung lösen. Die Biegefestigkeit und die Druckfestigkeiten wurden gemäß ISO 4049 bestimmt. Der Volumenschrumpf wurde aus den archimedisch bestimmten Dichten und Volumina der unpolymerisierten und der polymerisierten Zubereitungen errechnet.

Die Monomere bzw. Monomermischungen gemäß Komponente (a) wiesen alle eine Viskosität kleiner als 8 Pas auf. Die Viskosität wurde kraftgesteuert bestimmt und die Epoxide bzw. die Epoxidmischungen gemäß Komponente (a) im Meßbereich von 5-500 Pa als Newtonsch'sche Flüssigkeiten erkannt.

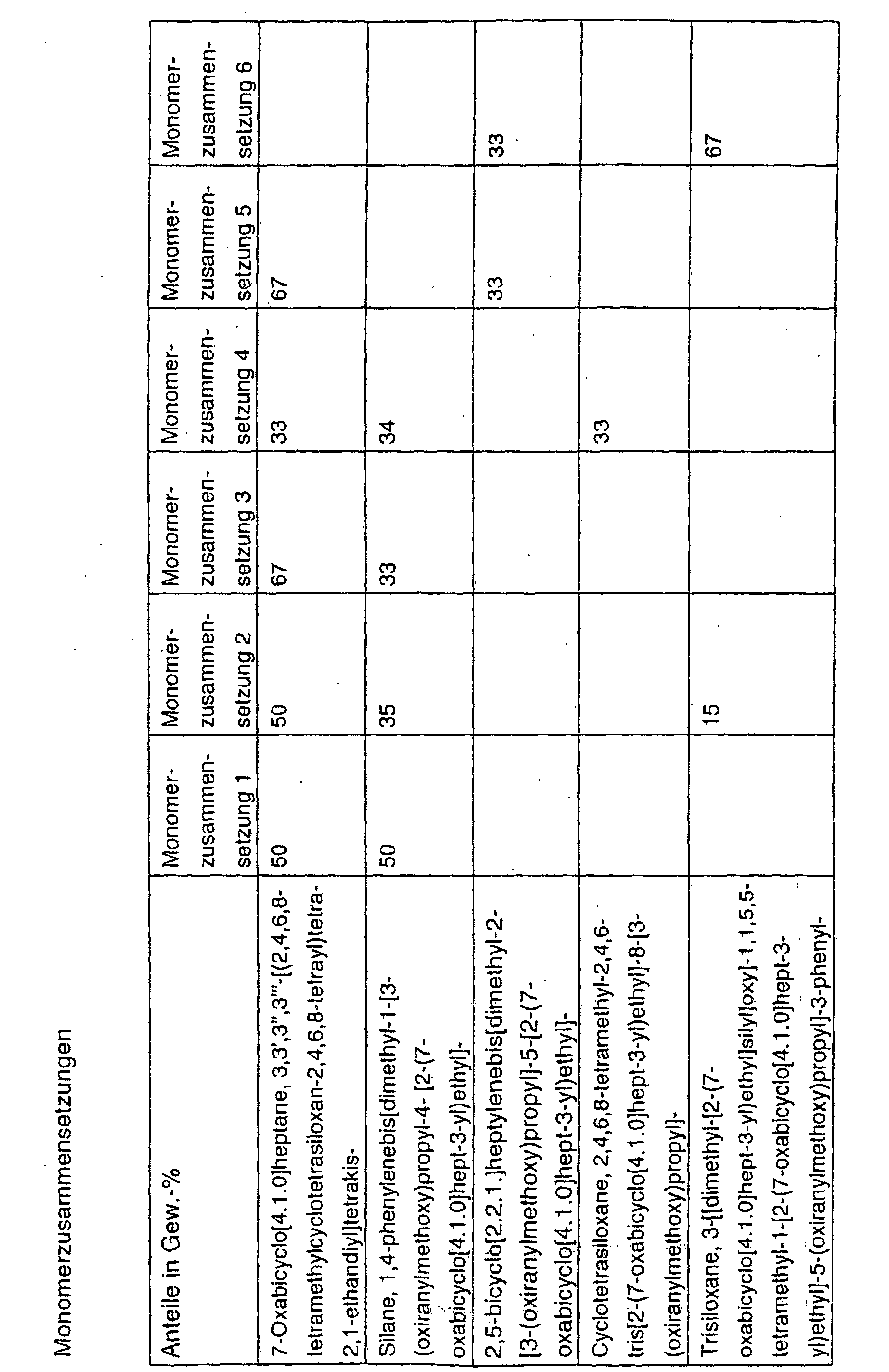

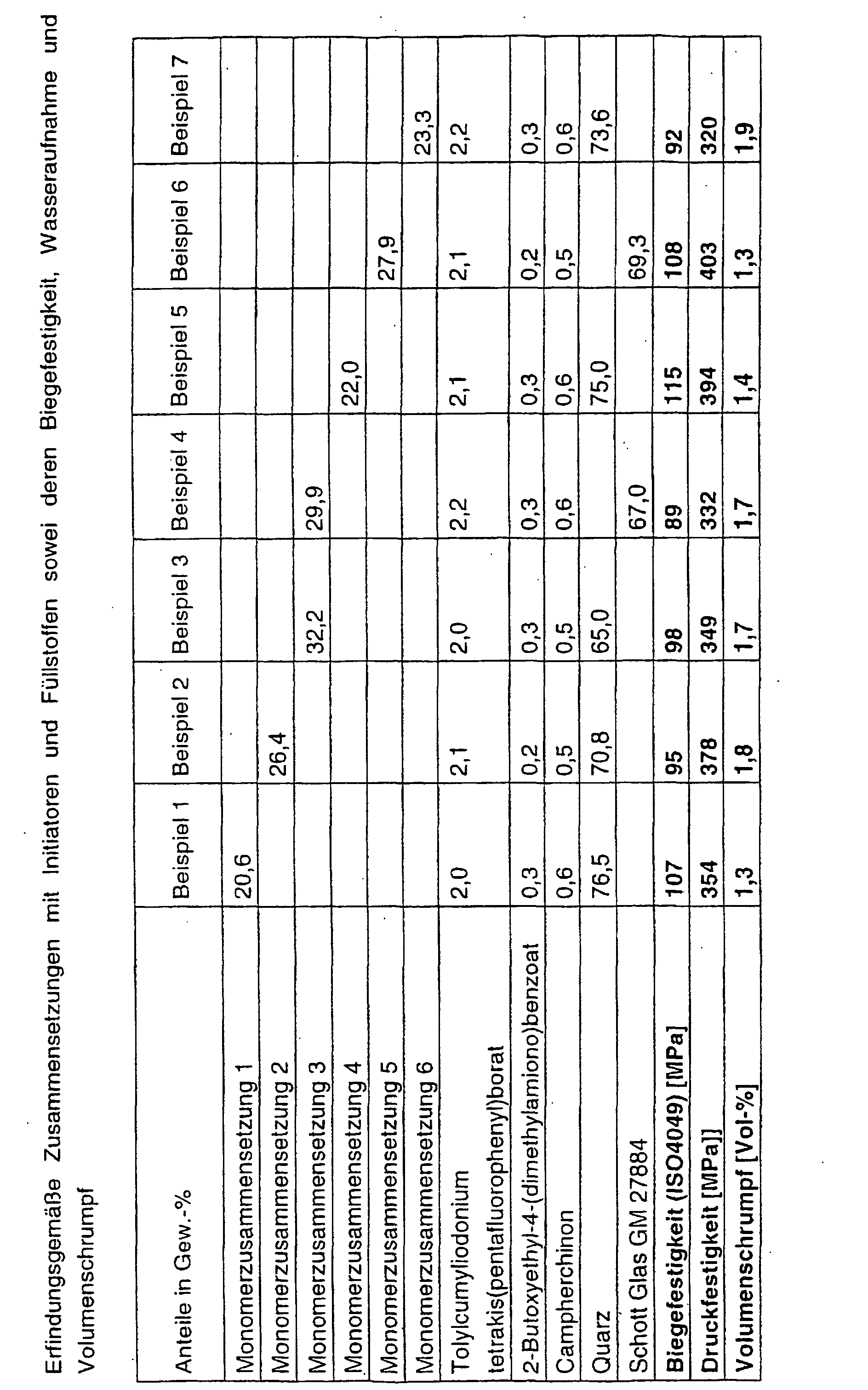

## Patentansprüche

1. Polymerisierbare Zubereitung, enthaltend:
(a) 1 bis 80 Gew.-% eines Epoxids oder eines Gemisches von Epoxiden der allgemeinen Formel: 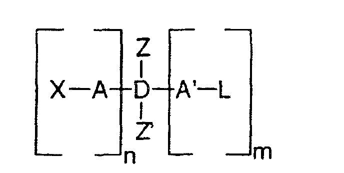 wobei
A, A' unabhängig voneinander einen unverzweigten oder verzweigten aliphatischen, cycloaliphatischen oder aromatischen Rest oder eine Kombination dieser Reste mit 0 bis 16 C-Atomen bedeuten, wobei ein oder mehrere C-Atome durch O, O(C=O), C=O, Si, N, S ersetzt sein können,
X einen unverzweigten oder verzweigten aliphatischen oder aromatischen Rest oder eine Kombination dieser Reste 3 bis 16 C-Atomen bedeutet, wobei ein oder mehrere C-Atome durch O, O(C=O), (C=O), Si, N, S ersetzt sein können und eine aliphatische end- oder mittelständige Epoxid-Gruppe enthalten ist,
L einen unverzweigten oder verzweigten aliphatischen, cycloaliphatischen oder aromatischen Rest oder eine Kombination dieser Reste mit 6 bis 30 C-Atomen bedeutet, wobei ein oder mehrere C-Atome durch O, O(C=O), (C=O), Si, N, S ersetzt sein können und eine cycloaliphatische Epoxidgruppe enthalten ist,
Z, Z' unabhängig voneinander H oder einen unverzweigten oder verzweigten aliphatischen, cycloaliphatischen oder aromatischen Rest oder eine Kombination dieser Reste mit 0 bis 10 C-Atomen bedeuten, wobei ein oder mehrere C-Atome durch O, O(C=O), (C=O), Si, N, S ersetzt sein können,
D einen unverzweigten oder verzweigten aliphatischen, cycloaliphatischen oder aromatischen Rest oder eine Kombination dieser Reste mit 1 bis 55 C-Atomen bedeutet, wobei mindestens ein C-Atom durch SiGG', SiG oder Si ersetzt ist und ein oder mehrere C-Atome durch O, O(C=O), (C=O), N oder S ersetzt sein können,
G, G' unabhängig voneinander, einen unverzweigten oder verzweigten aliphatischen, cycloaliphatischen oder aromatischen Rest oder eine Kombination dieser Reste mit 1 bis 15 C-Atomen bedeuten, wobei ein oder mehrere C-Atome durch O, O(C=O), (C=O), Si, N, S ersetzt sein können,
n und m unabhängig voneinander 1, 2, 3 oder 4 bedeuten,
wobei die Molmasse des Epoxids oder die mittlere Molmasse des Gemisches von Epoxiden 360 bis 3000 g/mol beträgt,
(b) 0 bis 80 Gew.-% eines Epoxids oder eines Gemisches von Epoxiden, die von (a) verschieden sind,
(c) 3 bis 85 Gew.-% Füllstoffe,
(d) 0,01 bis 25 Gew.-% Initiatoren, Verzögerer und/oder Beschleuniger,
(e) 0 bis 25 Gew.-% Hilfsstoffe,
wobei die Prozentangaben jeweils auf das Gesamtgewicht der Zubereitung bezogen sind.

2. Polymerisierbare Zubereitung nach Anspruch 1, enthaltend:
(a) 3 bis 50 Gew.-% eines Epoxids oder eines Gemisches von Epoxiden der allgemeinen Formel: 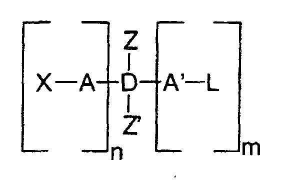 wobei
A, A' unabhängig voneinander einen unverzweigten oder verzweigten aliphatischen, cycloaliphatischen oder aromatischen Rest oder eine Kombination dieser Reste mit 0 bis 12 C-Atomen bedeuten, wobei ein oder mehrere C-Atome durch O, O(C=O), C=O, Si, N, S ersetzt sein können,
X einen unverzweigten oder verzweigten aliphatischen oder aromatischen Rest oder eine Kombination dieser Reste mit 3 bis 14 C-Atomen bedeuten, wobei ein oder mehrere C-Atome durch O, O(C=O), (C=O), Si, N, S ersetzt sein können und und eine aliphatische end- oder mittelständige Epoxid-Gruppe enthalten ist,
L einen unverzweigten oder verzweigten aliphatischen, cycloaliphatischen oder aromatischen Rest oder eine Kombination dieser Reste mit 6 bis 20 C-Atomen bedeutet, wobei ein oder mehrere C-Atome durch O, O(C=O), (C=O), Si, N, S ersetzt sein können und eine cycloaliphatische Epoxidgruppe enthalten ist,
Z, Z' unabhängig voneinander H oder einen unverzweigten oder verzweigten aliphatischen, cycloaliphatischen oder aromatischen Rest oder eine Kombination dieser Reste mit 0 bis 10 C-Atomen bedeuten, wobei ein oder mehrere C-Atome durch O, O(C=O), (C=O), Si ersetzt sein können,
D einen unverzweigten oder verzweigten aliphatischen, cycloaliphatischen oder aromatischen Rest oder eine Kombination dieser Reste mit 1 bis 50 C-Atomen bedeutet, wobei mindestens ein C-Atom durch SiGG', SiG oder Si ersetzt ist und ein oder mehrere C-Atome durch O, O(C=O), (C=O) ersetzt sein können,
G, G' unabhängig voneinander einen unverzweigten oder verzweigten aliphatischen, cycloaliphatischen oder aromatischen Rest oder eine Kombination dieser Reste mit 1 bis 8 C-Atomen bedeuten, wobei ein oder mehrere C-Atome durch O, O(C=O), (C=O), Si ersetzt sein können,
n und m unabhängig voneinander 1, 2, 3 oder 4 bedeuten,
wobei die Molmasse des Epoxids oder die mittlere Molmasse des Gemisches von Epoxiden 360 bis 2500 g/mol beträgt,
(b) 0 bis 60 Gew.-% eines Epoxids oder eines Gemisches von Epoxiden, die von (a) verschieden sind,
(c) 15 bis 85 Gew.-% Füllstoffe,
(d) 0,01 bis 20 Gew.-% Initiatoren, Verzögerer und/oder Beschleuniger,
(e) 0 bis 25 Gew.-% Hilfsstoffe,
wobei die Prozentangaben jeweils auf das Gesamtgewicht der Zubereitung bezogen sind.

3. Polymerisierbare Zubereitung nach einem der Ansprüche 1 oder 2, wobei sie als Komponente (a) eines oder mehrere der folgenden Epoxide enthält: 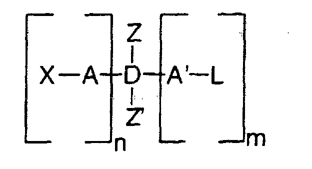 wobei
A, A' unabhängig voneinander einen unverzweigten oder verzweigten aliphatischen, cycloaliphatischen oder aromatischen Rest oder eine Kombination dieser Reste mit 0 bis 12 C-Atomen bedeuten, wobei ein oder mehrere C-Atome durch O, O(C=O), C=O, Si, N, S ersetzt sein können,
X einen unverzweigten oder verzweigten aliphatischen Rest oder eine Kombination dieser Reste mit 3 bis 14 C-Atomen bedeutet, wobei ein oder mehrere C-Atome durch O, O(C=O), (C=O), Si, N, S ersetzt sein können und mindestens eine endständige aliphatische Epoxidgruppe enthalten ist,
L einen unverzweigten oder verzweigten aliphatischen, cycloaliphatischen Rest oder eine Kombination dieser Reste mit 7 bis 15 C-Atomen bedeutet, in dem ein oder mehrere C-Atome durch O, O(C=O), (C=O), Si, N, S ersetzt sein können, und eine cycloaliphatische Epoxidgruppe der nachfolgenden Formel enthalten ist, 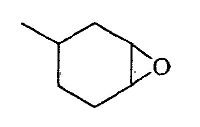 D einen unverzweigten oder verzweigten aliphatischen, cycloaliphatischen oder aromatischen Rest oder eine Kombination dieser Reste mit 1 bis 50 C-Atomen bedeutet, wobei mindestens ein C-Atom durch SiGG', SiG oder Si ersetzt ist und ein oder mehrere C-Atome durch O, O(C=O), (C=O) ersetzt sein können,
G, G' unabhängig voneinander einen unverzweigten oder verzweigten aliphatischen oder aromatischen Rest oder eine Kombination dieser Reste mit 1 bis 8 C-Atomen bedeuten, wobei ein oder mehrere C-Atome durch O, O(C=O), (C=O), Si ersetzt sein können,
n und m unabhängig voneinander 1, 2, 3 oder 4 bedeuten,
wobei die Molmasse des Epoxids oder die mittlere Molmasse des Gemisches von Epoxiden 360 bis 2500 g/mol beträgt.

4. Polymerisierbare Zubereitung nach einem der Ansprüche 1 bis 3, wobei sie als Komponente (a) eines oder mehrere der folgenden Epoxide enthält: 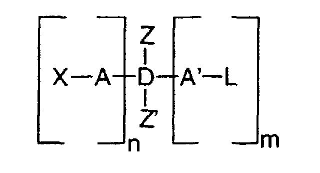 wobei
A, A' unabhängig voneinander einen unverzweigten oder verzweigten aliphatischen, cycloaliphatischen oder aromatischen Rest oder eine Kombination dieser Reste mit 0 bis 10 C-Atomen bedeuten, wobei ein oder mehrere C-Atome durch O, O(C=O), C=O, Si ersetzt sein können,
X einen verzweigten oder unverzweigten aliphatischen Rest mit 3 bis 14 C-Atomen bedeutet, in dem ein oder mehrere C-Atome durch O, O(C=O), (C=O), Si ersetzt sein können und mindestens eine endständige aliphatische Epoxidgruppe enthalten ist,
L einen unverzweigten oder verzweigten aliphatischen oder cycloaliphatischen Rest oder eine Kombination dieser Reste mit 7 bis 15 C-Atomen bedeutet, in dem ein oder mehrere C-Atome durch O, O(C=O), (C=O), Si ersetzt sein können, und eine cycloaliphatische Epoxidgruppe der nachfolgenden Formel enthalten ist, 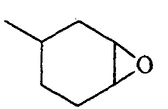 D einen unverzweigten oder verzweigten aliphatischen, cycloaliphatischen oder aromatischen Rest oder eine Kombination dieser Reste mit 1 bis 50 C-Atomen bedeutet, wobei mindestens ein C-Atom durch SiGG', SiG oder Si ersetzt ist und ein oder mehrere C-Atome durch O, O(C=O), (C=O) ersetzt sein können,
G, G' unabhängig voneinander einen unverzweigten oder verzweigten aliphatischen oder aromatischen Rest oder eine Kombination dieser Reste mit 1 bis 8 C-Atomen bedeuten,
n und m unabhängig voneinander 1, 2, 3 oder 4 bedeuten und m+n 2 bis 6 ergeben,
wobei die Molmasse des Epoxids oder die mittlere Molmasse des Gemisches von Epoxiden 360 bis 2500 g/mol beträgt.

5. Polymerisierbare Zubereitung nach einem der Ansprüche 1 bis 4, wobei Komponente (a) einen der folgenden Bestandteile D aufweist, der über das Si-Atom an die Bestandteile A und/oder A' angebunden ist
i. Si
ii. SiG
iii. SiGG' 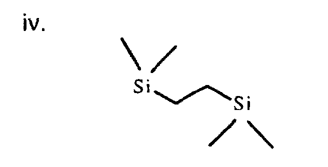 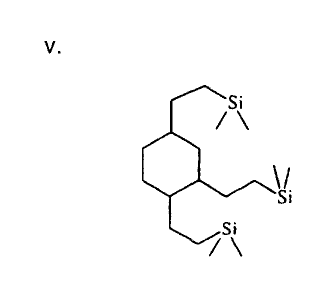 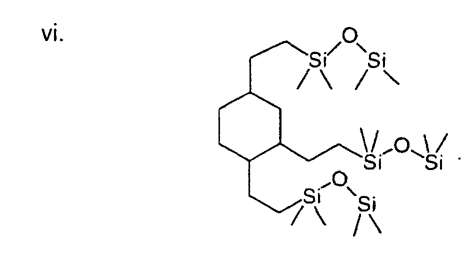 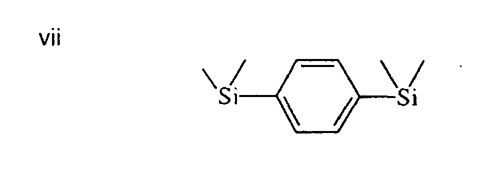 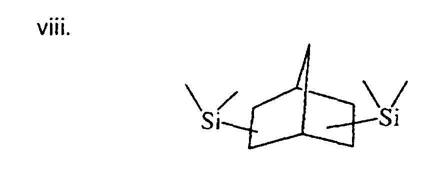 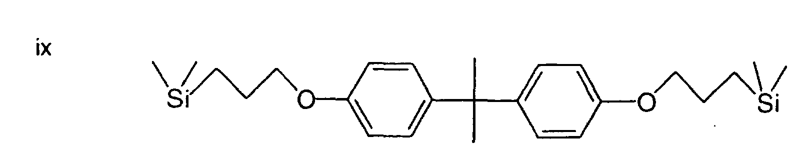 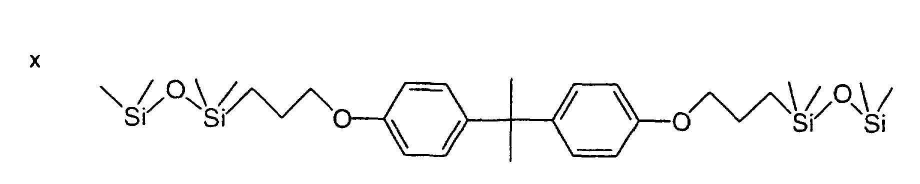 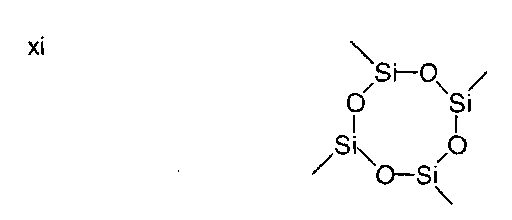 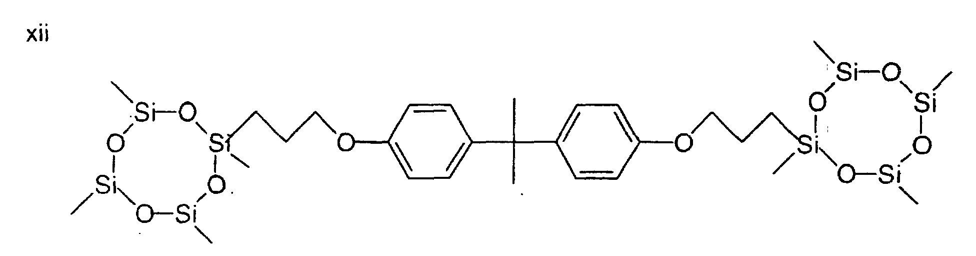 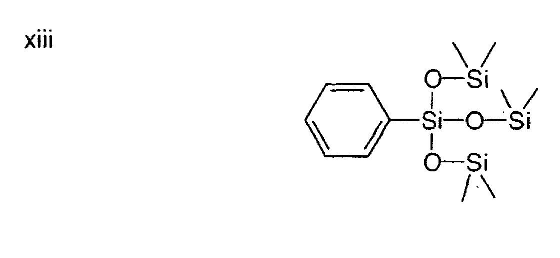
wobei G, G' unabhängig voneinander einen unverzweigten oder verzweigten aliphatischen, cycloaliphatischen oder aromatischen Rest oder eine Kombination dieser Reste mit 1 bis 8 C-Atomen bedeuten, wobei ein oder mehrere C-Atome durch O, O(C=O), (C=O), Si ersetzt sein können.

6. Polymerisierbare Zubereitung nach einem der Ansprüche 1 bis 5, wobei sie als Komponente (a) eines oder mehrere der folgenden Epoxide enthält:
i. Silane, 1,2-ethylene-[1,2-dimethyl-1-[3-(oxiranylmethoxy)propyl]-2-[2-(7-oxabicyclo[4.1.0]hept-3-yl)ethyl]- 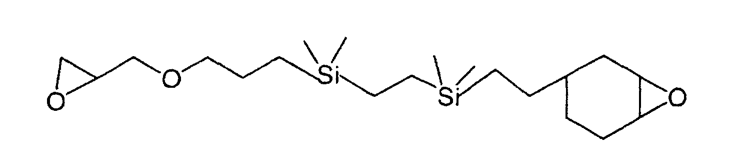
ii. Silane, 1,1',1"-(1,2,4-cyclohexylene-1-(dimethyl[3-(oxiranylmethoxy)propy)])-2,4-bis[2-(7-oxabicyclo[4.1.0]hept-3-yl)ethyl])- 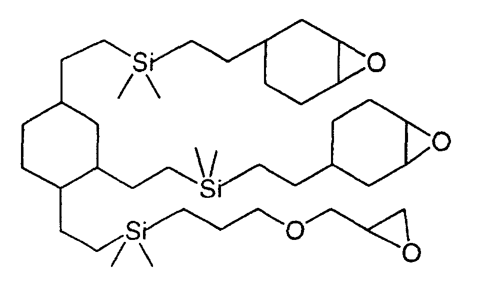
iii. Disiloxane, 1,1',1"-(1,2,4-cyclohexanetriyltri-2,1-ethanediyl)-1,1,3,3-tetramethyl-3-(oxiranylmethoxy)propylbis[1,1,3,3-tetramethyl-2-(7-oxabicyclo[4.1.0]hept-3-yl)ethyl]- 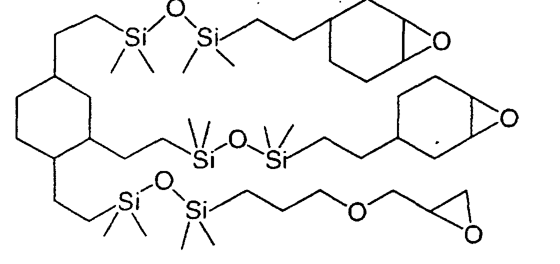
iv Silane, 1,4-phenylenebis[dimethyl-1-[3-(oxiranylmethoxy)propyl-4- [2-(7-oxabicyclo[4.1.0]hept-3-yl)ethyl]- 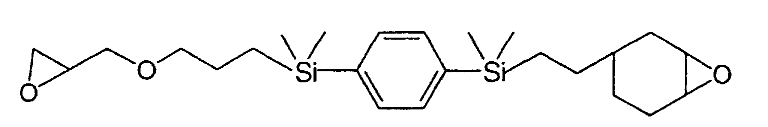
v. Silane, 2,5-bicyclo[2.2.1.]heptylenebis[dimethyl-2-[3-(oxiranylmethoxy)-propyl]-5-[2-(7-oxabicyclo[4.1.0]hept-3-yl)ethyl]- 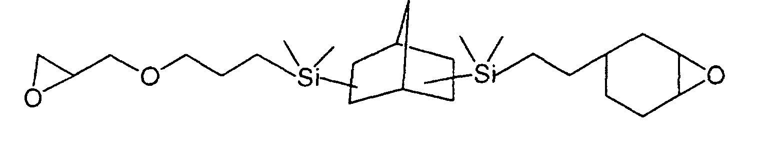
vi. Silane, 1,1'-[bis(4,4'-hydroxyphenyl-2,2-propane-3,1-propanediyl)]-1-dimetyl[3-(oxiranylmethoxy)propyl]-1'-dimethyl[2-(7-oxabicyclo[4.1.0]hept-3-yl)ethyl]- 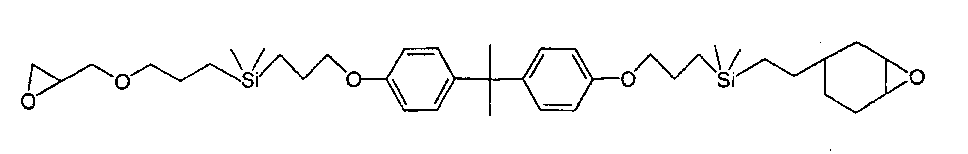
vii. Siloxane, 1,1'-[bis(4,4'-hydroxyphenyl-2,2-propane-3,1-propanediyl)-1,1,3,3-tetrametyl]-1-[3-(oxiranylbutyl]-1'-[2-(7-oxabicyclo[4.1.0]hept-3-yl)ethyl]- 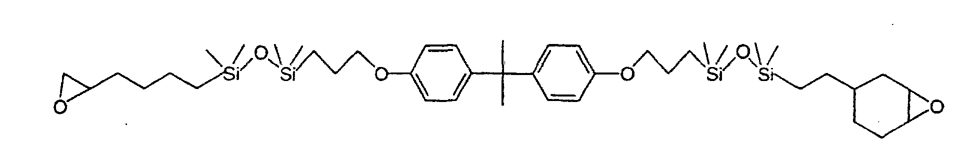
viii. Cyclotetrasiloxane, 2,4,6,8-tetramethyl-2,4,6-tris[2-(7-oxabicyclo[4.1.0]hept-3-yl)ethyl]-8-[3-(oxiranylmethoxy)propyl]- 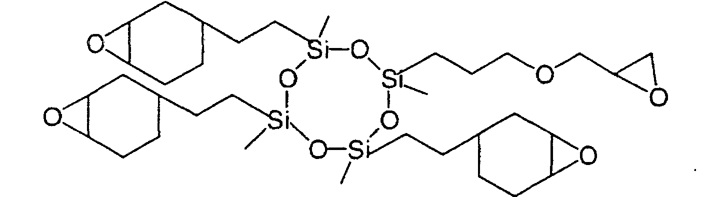
ix. Cyclotetrasiloxane, 2,4,6,8-tetramethyl-2,4,6-tris[2-(7-oxabicyclo[4.1.0]hept-3-yl)ethyl]-8-[4-oxiranylbutyl]- 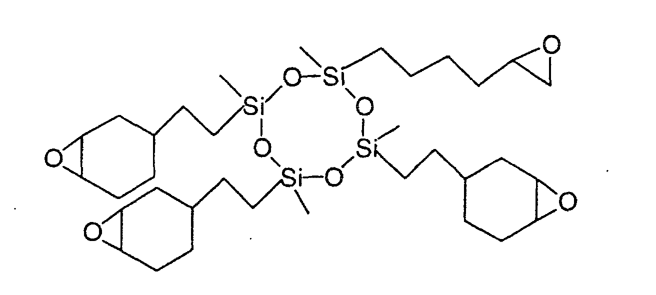
x. Cyclotetrasiloxane, 1,1'-[bis(4,4'-hydroxyphenyl-2,2-propane-3,1-propanediyl)]bis[2,4,6,8-tetramethyl-4,6-bis[3-(oxiranylmethoxy)propyl]-8-[; (7-oxabicyclo[4.1.0]hept-3-yl)ethyl]- 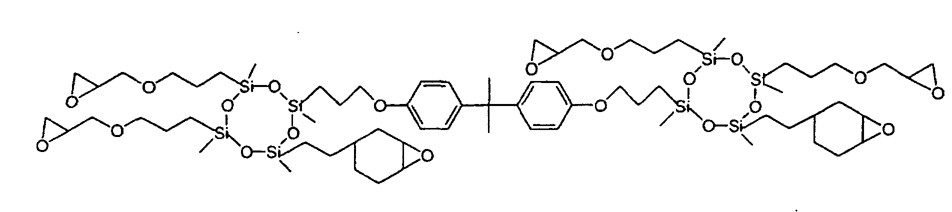
xi. Trisiloxane, 3-[[dimethyl-[2-(7-oxabicyclo[4.1.0]hept-3-yl)ethyl)silyl]oxy]-1,1,5,5-tetramethyl-1-[2-(7-oxabicyclo[4.1.0]hept-3-yl)ethyl]-5-(oxiranylmethoxy)propyl]-3-phenyl- 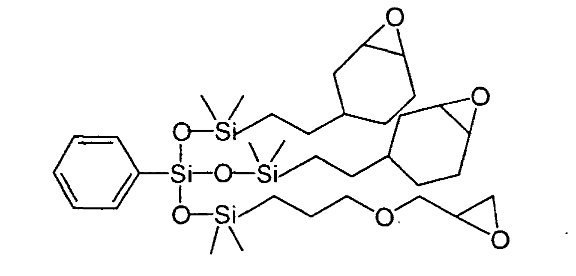
xii. Trisiloxane, 3-[[dimethyl-[2-(7-oxabicyclo[4.1.0]hept-3-yl)ethyl]silyl]oxy]-1,1,5,5-tetramethyl-1-[2-(7-oxabicyclo[4.1.0]hept-3-yl)ethyl]-5-[2-(oxiranylethyl)]-3-phenyl-

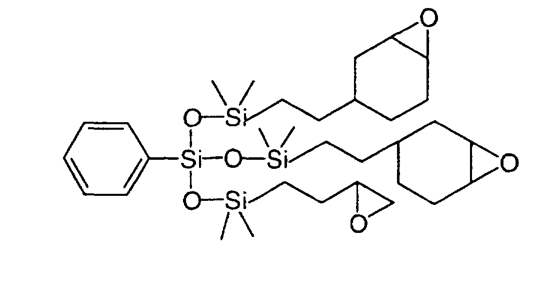

7. Polymerisierbare Zubereitung nach einem der Ansprüche 1 bis 6, wobei sie als Füllstoffe gemäß Komponente (c) Quarz, gemahlene Gläser, Kieselgele und/oder Kieselsäuren, deren Granulate und/oder gemahlene Kunststoffe enthält.

8. Polymerisierbare Zubereitung nach einem der Ansprüche 1 bis 7, wobei sie als Initiatoren Lewis-Säuren und/oder Brönsted-Säuren oder Verbindungen, aus denen durch Bestrahlen mit UV-Licht, sichtbarem Licht, Druck und/oder Wärme oder durch chemische Reaktion solche Säuren entstehen, enthält.

9. Polymerisierbare Zubereitung nach einem der Ansprüche 1 bis 8, wobei sie als Beschleuniger hydroxyfunktionalisierte Epoxide enthält.

10. Polymerisierbare Zubereitung nach einem der Ansprüche 1 bis 9, wobei sie als Hilfsstoffe Verdünnungsmittel, Stabilisatoren, Inhibitoren und/oder Pigmente enthält.

11. Polymerisierbare Zubereitung nach einem der Ansprüche 1 bis 10, enthaltend
A mindestens eine Basispaste, enthaltend Epoxide oder Gemische von Epoxiden der Komponenten (a) und (b), einen Teil oder den gesamten Anteil der Füllstoffe der Komponente (c), gegebenenfalls Verzögerer und/oder Beschleuniger gemäß Komponente (d), und gegebenenfalls Hilfsstoffe der Komponente (e), sowie räumlich getrennt hiervon
B mindestens eine Katalysatorpaste, enthaltend mindestens einen Initiator gemäß Komponente (d) gegebenenfalls einen Teil der Füllstoffe der Komponente (c) und gegebenenfalls Hilfsstoffe gemäß Komponente (e),
wobei Basis- und Katalysatorpaste zum Erhalt der polymerisierbaren Zubereitung miteinander zur Reaktion gebracht werden.

12. Verwendung der polymerisierbaren Zubereitung nach einem der Ansprüche 1 bis 11 als Beschichtungsmittel und/oder zum Verkleben von Substraten.

13. Verwendung der polymerisierbaren Zubereitung nach einem der Ansprüche 1 bis 11 als Dentalmasse.

14. Behältnis, insbesondere Kartusche oder Mischkapsel, enthaltend eine polymerisierbare Zubereitung nach einem der Ansprüche 1 bis 11.

15. Ausbringvorrichtung, enthaltend eine polymerisierbare Zubereitung nach einem der Ansprüche 1 bis 11.

## Claims

1. A polymerizable preparation comprising:
(a) 1 to 80 weight% of an epoxide or a mixture of epoxides of the general formula: 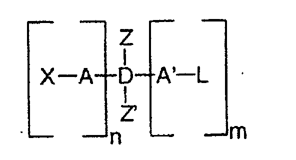 in which
A and A' represent, independently of one another, an unbranched or branched aliphatic, cycloaliphatic or aromatic residue or a combination of these residues with 0 to 16 carbon atoms, in which one or more carbon atoms can be replaced by O, O(C=O), C=O, Si, N or S,
X represents an unbranched or branched aliphatic or aromatic residue or a combination of these residues with 3 to 16 carbon atoms, in which one or more carbon atoms can be replaced by 0, O(C=O), (C=O), Si, N or S and an aliphatic terminal or centrally located epoxide group is present,
L represents an unbranched or branched aliphatic, cycloaliphatic or aromatic residue or a combination of these residues with 6 to 30 carbon atoms, in which one or more carbon atoms can be replaced by O, O(C=O), (C=O), Si, N or S and a cycloaliphatic epoxide group is present,
Z and Z' represent, independently of one another, H or an unbranched or branched aliphatic, cycloaliphatic or aromatic residue or a combination of these residues with 0 to 10 carbon atoms, in which one or more carbon atoms can be replaced by O, O(C=O), (C=O), Si, N or S,
D represents an unbranched or branched aliphatic, cycloaliphatic or aromatic residue or a combination of these residues with 1 to 55 carbon atoms, in which at least one carbon atom is replaced by SiGG', SiG or Si and one or more carbon atoms can be replaced by O, O(C=O), (C=O), N or S,
G and G' represent, independently of one another, an unbranched or branched aliphatic, cycloaliphatic or aromatic residue or a combination of these residues with 1 to 15 carbon atoms, in which one or more carbon atoms can be replaced by O, O(C=O), (C=O), Si, N or S,
n and m represent, independently of one another, 1, 2, 3 or 4,
in which the molar mass of the epoxide or the average molar mass of the mixture of epoxides is 360 to 3 000 g/mol,
(b) 0 to 80 weight% of an epoxide or a mixture of epoxides which are different from (a),
(c) 3 to 85 weight% of fillers,
(d) 0.01 to 25 weight% of initiators, retarders and/or accelerators,
(e) 0 to 25 weight% of auxiliaries,
in which the percentages are in each case with reference to the total weight of the preparation.

2. The polymerizable preparation according to claim 1, comprising:
(a) 3 to 50 weight% of an epoxide or a mixture of epoxides of the general formula: 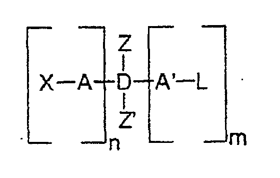 in which
A and A' represent, independently of one another, an unbranched or branched aliphatic, cycloaliphatic or aromatic residue or a combination of these residues with 0 to 12 carbon atoms, in which one or more carbon atoms can be replaced by 0, O(C=O), C=O, Si, N or S,
X represents an unbranched or branched aliphatic or aromatic residue or a combination of these residues with 3 to 14 carbon atoms, in which one or more carbon atoms can be replaced by 0, O(C=O), (C=O), Si, N or S and an aliphatic terminal or centrally located epoxide group is present,
L represents an unbranched or branched aliphatic, cycloaliphatic or aromatic residue or a combination of these residues with 6 to 20 carbon atoms, in which one or more carbon atoms can be replaced by O, O(C=O), (C=O), Si, N or S and a cycloaliphatic epoxide group is present,
Z and Z' represent, independently of one another, H or an unbranched or branched aliphatic, cycloaliphatic or aromatic residue or a combination of these residues with 0 to 10 carbon atoms, in which one or more carbon atoms can be replaced by O, O(C=O), (C=O) or Si,
D represents an unbranched or branched aliphatic, cycloaliphatic or aromatic residue or a combination of these residues with 1 to 50 carbon atoms, in which at least one carbon atom is replaced by SiGG', SiG or Si and one or more carbon atoms can be replaced by O, O(C=O) or (C=O),
G and G' represent, independently of one another, an unbranched or branched aliphatic, cycloaliphatic or aromatic residue or a combination of these residues with 1 to 8 carbon atoms, in which one or more carbon atoms can be replaced by O, O(C=O), (C=O) or Si,
n and m represent, independently of one another, 1, 2, 3 or 4,
in which the molar mass of the epoxide or the average molar mass of the mixture of epoxides is 360 to 2 500 g/mol,
(b) 0 to 60 weight% of an epoxide or a mixture of epoxides which are different from (a),
(c) 15 to 85 weight% of fillers,
(d) 0.01 to 20 weight% of initiators, retarders and/or accelerators,
(e) 0 to 25 weight% of auxiliaries,
in which the percentages are in each case with reference to the total weight of the preparation.

3. The polymerizable preparation according to claim 1 or 2, comprising, as component (a), one or more of the following epoxides: 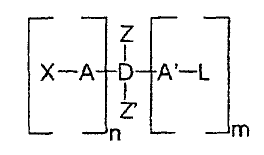 in which
A and A' represent, independently of one another, an unbranched or branched aliphatic, cycloaliphatic or aromatic residue or a combination of these residues with 0 to 12 carbon atoms, in which one or more carbon atoms can be replaced by O, O(C=O), C=O, Si, N or S,
X represents an unbranched or branched aliphatic residue or a combination of these residues with 3 to 14 carbon atoms, in which one or more carbon atoms can be replaced by O, O(C=O), (C=O) , Si, N or S and at least one terminal aliphatic epoxide group is present,
L represents an unbranched or branched aliphatic or cycloaliphatic residue or a combination of these residues with 7 to 15 carbon atoms, in which one or more carbon atoms can be replaced by O, O(C=O), (C=O), Si, N or S and a cycloaliphatic epoxide group of the following formula is present, 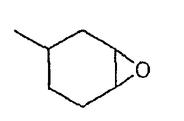 D represents an unbranched or branched aliphatic, cycloaliphatic or aromatic residue or a combination of these residues with 1 to 50 carbon atoms, in which at least one carbon atom is replaced by SiGG', SiG or Si and one or more carbon atoms can be replaced by O, O(C=O) or (C=O),
G and G' represent, independently of one another, an unbranched or branched aliphatic or aromatic residue or a combination of these residues with 1 to 8 carbon atoms, in which one or more carbon atoms can be replaced by 0, O(C=O), (C=O) or Si,
n and m represent, independently of one another, 1, 2, 3 or 4,
in which the molar mass of the epoxide or the average molar mass of the mixture of epoxides is 360 to 2 500 g/mol.

4. The polymerizable preparation according to one of the claims 1 to 3, comprising, as component (a), one or more of the following epoxides: 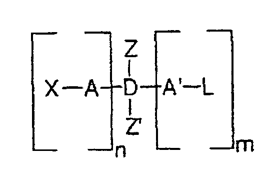 in which
A and A' represent, independently of one another, an unbranched or branched aliphatic, cycloaliphatic or aromatic residue or a combination of these residues with 0 to 10 carbon atoms, in which one or more carbon atoms can be replaced by O, O(C=O), C=O or Si,
X represents a branched or unbranched aliphatic residue with 3 to 14 carbon atoms, in which one or more carbon atoms can be replaced by O, O(C=O), (C=O) or Si and at least one terminal aliphatic epoxide group is present,
L represents an unbranched or branched aliphatic or cycloaliphatic residue or a combination of these residues with 7 to 15 carbon atoms, in which one or more carbon atoms can be replaced by O, O(C=O), (C=O) or Si and a cycloaliphatic epoxide group of the following formula is present, 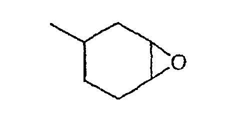 D represents an unbranched or branched aliphatic, cycloaliphatic or aromatic residue or a combination of these residues with 1 to 50 carbon atoms, in which at least one carbon atom is replaced by SiGG', SiG or Si and one or more carbon atoms can be replaced by O, O(C=O) or (C=O) ,
G and G' represent, independently of one another, an unbranched or branched aliphatic or aromatic residue or a combination of these residues with 1 to 8 carbon atoms,
n and m represent, independently of one another, 1, 2, 3 or 4 and m+n amount to 2 to 6,
in which the molar mass of the epoxide or the average molar mass of the mixture of epoxides is 360 to 2 500 g/mol.

5. The polymerizable preparation according to one of the claims 1 to 4, in which component (a) contains one of the following constituents D, which is bonded to the constituents A and/or A' via the silicon atom:
i. Si
ii. SiG
iii. SiGG' 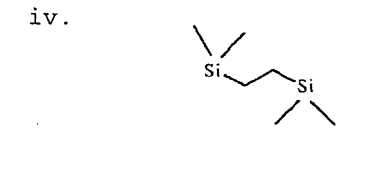 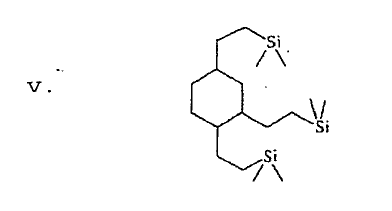 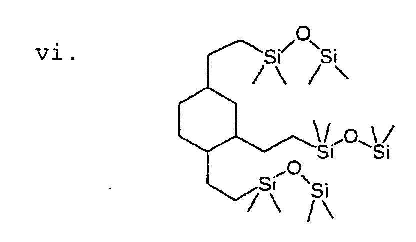 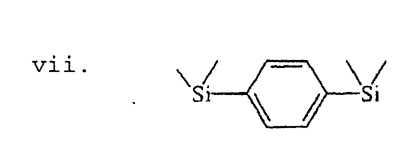 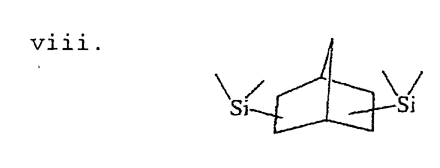 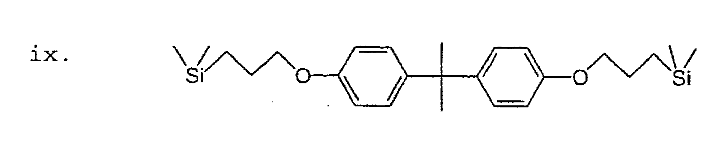 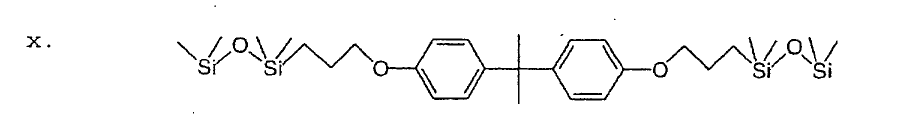 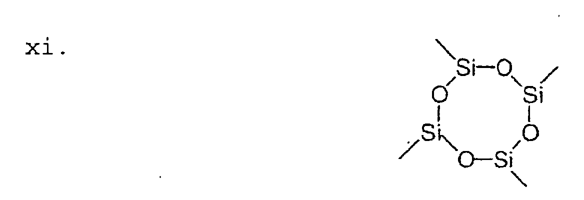 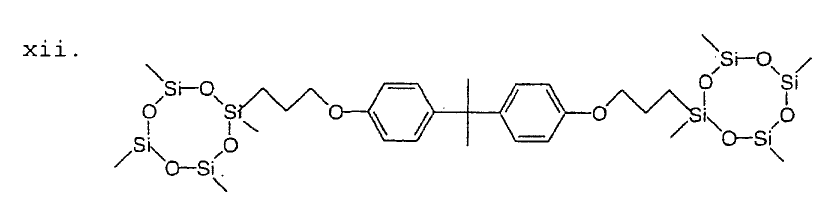 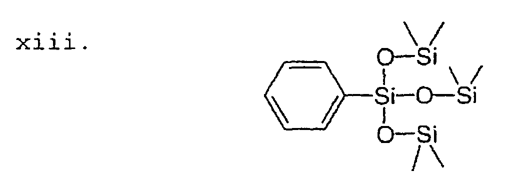
in which G and G' represent, independently of one another, an unbranched or branched aliphatic, cycloaliphatic or aromatic residue or a combination of these residues with 1 to 8 carbon atoms, in which one or more carbon atoms can be replaced by O, O(C=O), (C=O) or Si.

6. The polymerizable preparation according to one of the claims 1 to 5, comprising, as component (a), one or more of the following epoxides:
i. Silane, 1,2-ethylene-[2,2-dimethyl-1-[3-(oxiranylmethoxy)propyl]-2-[2-(7-oxabicyclo[4.1.0]hept-3-yl)ethyl]- 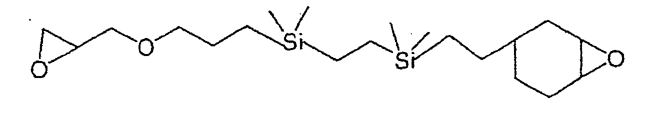
ii. Silane, 1,1',1"-(1,2,4-cyclohexylene-1-(dimethyl-[3-(oxiranylmethoxy)propyl])-2,4-bis[2-(7-oxabicyclo[4.1.0]hept-3-yl)ethyl])- 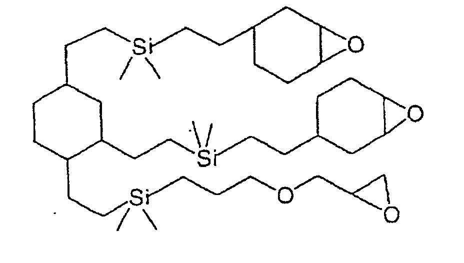
iii. Disiloxane, 1,1',1"-(1,2,4-cyclohexanetriyltri-2,1-ethanediyl)-1,1,3,3-tetramethyl-3-(oxiranylmethoxy)propylbis[1,1,3,3-tetramethyl-2-(7-oxabicyclo[4.1.0]hept-3-yl)ethyl]- 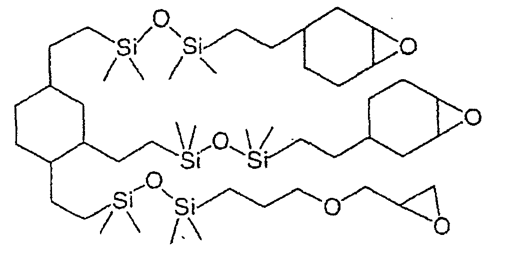
iv. Silane, 1,4-phenylenebis[dimethyl-1-[3-(oxiranylmethoxy)propyl-4-[2-(7-oxabicyclo[4.1.0]hept-3-yl)ethyl)- 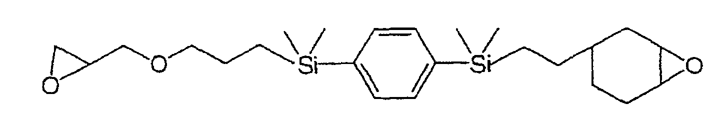
v. Silane, 2,5-bicyclo[2.2.1.]heptylenebis[dimethyl-2-[3-(oxiranylmethoxy)propyl]-5-[2-(7-oxabicyclo-[4.1.0]hept-3-yl)ethyl]- 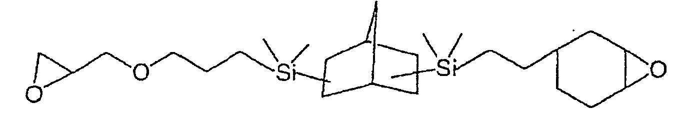
vi. Silane, 1,1'-[bis(4,4'-hydroxyphenyl-2,2-propane-3,1-propanediyl)]-1-dimetyl[3-(oxiranylmethoxy)-propyl]-1'-dimethyl[2-(7-oxabicyclo[4.1.0]hept-3-yl)ethyl]- 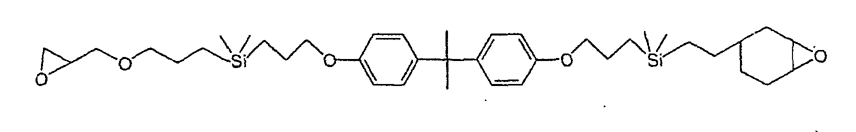
vii. Siloxane, 1,1'-[bis(4,4'-hydroxyphenyl-2,2-propane-3,1-propanediyl)-1,1,3,3-tetrametyl]-1-[3-(oxiranylbutyl]-1'-[2-(7-oxabicyclo[4.1.0]hept-3-yl)ethyl]- 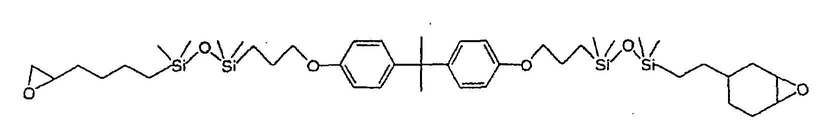
viii. Cyclotetrasiloxane, 2,4,6,8-tetramethyl-2,4,6-tris[2-(7-oxabicyclo[4.1.0]hept-3-yl)ethyl]-8-[3-(oxiranylmethoxy)propyl]- 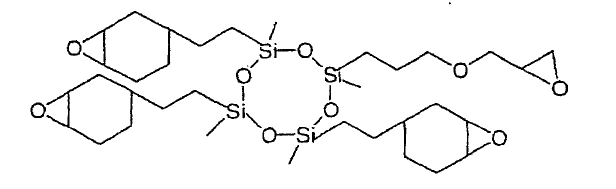
ix. Cyclotetrasiloxane, 2,4,6,8-tetramethyl-2,4,6-tris[2-(7-oxabicyclo[4.1.0]hept-3-yl)ethyl]-8-[4-oxiranylbutyl]- 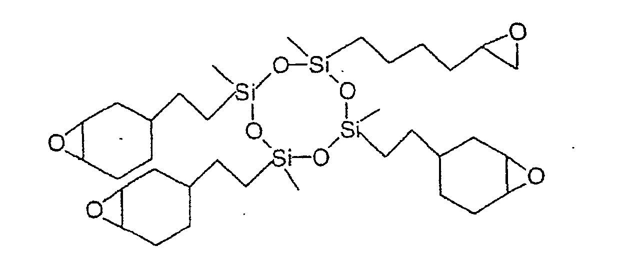
x. Cyclotetrasiloxane, 1,1'-[bis(4,4'-hydroxyphenyl-2,2-propane-3,1-propanediyl)]bis(2,4,6,8-tetramethyl-4,6-bis[3-(oxiranylmethoxy)propyl]-8-[2-(7-oxabicyclo[4.1.0]hept-3-yl)ethyl]- 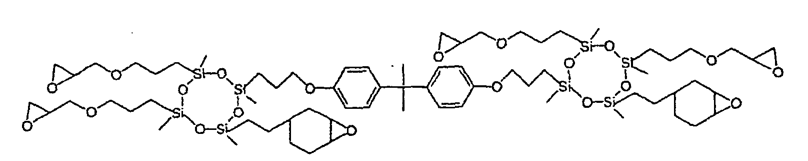
xi. Trisiloxane, 3-[[dimethyl-[2-(7-oxabicyclo-[4.1.0]hept-3-yl)ethyl]silyl]oxy]-1,1,5,5-tetramethyl-1-[2-(7-oxabicyclo[4.1.0]hept-3-yl)ethyl]-5-(oxiranylmethoxy)propyl]-3-phenyl- 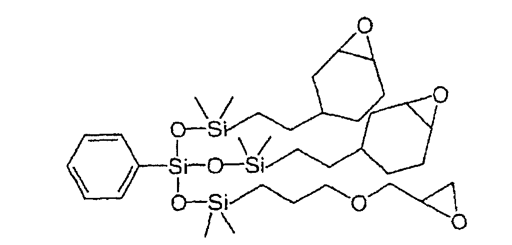
xii. Trisiloxane, 3-[[dimethyl-[2-(7-oxabicyclo-[4.1.0]hept-3-yl)ethyl]silyl]oxy]-1,1,5,5-tetramethyl-1-[2-(7-oxabicyclo[4.1.0]hept-3-yl)ethyl]-5-[2-(oxiranylethyl)]-3-phenyl-

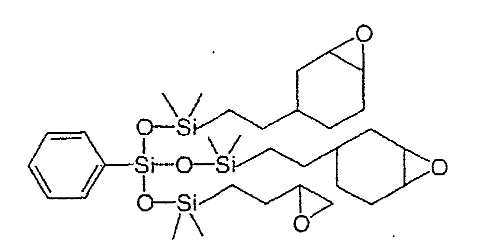

7. The polymerizable preparation according to one of the claims 1 to 6, comprising, as fillers according to component (c), quartz, ground glasses, silica gels and/or silicas, granulates thereof and/or ground plastics.

8. The polymerizable preparation according to one of the claims 1 to 7, comprising, as initiators, Lewis acids and/or Bronsted acids or compounds from which such acids arise by irradiation with UV light or visible light, pressure and/or heat or by chemical reaction.

9. The polymerizable preparation according to one of the claims 1 to 8, comprising, as accelerators, hydroxy-functionalized epoxides.

10. The polymerizable preparation according to one of the claims 1 to 9, comprising, as auxiliaries, diluents, stabilizers, inhibitors and/or pigments.

11. The polymerizable preparation according to one of the claims 1 to 10, comprising
A at least one base paste, comprising epoxides or mixtures of epoxides of component (a) and (b), a portion or the total amount of the fillers of component (c), optionally retarders and/or accelerators according to component (d), and optionally auxiliaries of component (e), and, spatially separated therefrom,
B at least one catalyst paste, comprising at least one initiator according to component (d), optionally a portion of the fillers of component (c) and optionally auxiliaries according to component (e),
in which base and catalyst paste are reacted with one another to obtain the polymerizable preparation.

12. Use of the polymerizable preparation according to one of the claims 1 to 11 as coating agent and/or for the cementing of substrates.

13. Use of the polymerizable preparation according to one of the claims 1 to 11 as dental material.

14. A container, in particular a cartridge or mixing capsule, comprising a polymerizable preparation according to one of the claims 1 to 11.

15. A dispensing device, comprising a polymerizable preparation according to one of the claims 1 to 11.

## Revendications

1. Préparation polymérisable contenant :
(a) 1 à 80 % en poids d'un époxyde ou d'un mélange d'époxydes de formule générale : 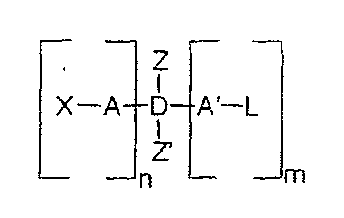 dans laquelle
A, A' représentent, indépendamment l'un de l'autre, un radical aliphatique ramifié ou non ramifié, cycloaliphatique ou aromatique ou une combinaison de ces radicaux ayant de 0 à 16 atomes de carbone, un ou plusieurs atomes de carbone pouvant être remplacés par 0, O(C=O), (C=O), Si, N, S,
X représente un radical aliphatique ramifié ou non ramifié ou aromatique ou une combinaison de ces radicaux ayant de 3 à 16 atomes de carbone, un ou plusieurs atomes de carbone pouvant être remplacés par O, O(C=O), (C=O), Si, N, S, et un groupe époxy aliphatique en milieu ou en bout de chaîne étant contenu,
L représente un radical aliphatique ramifié ou non ramifié, cycloaliphatique ou aromatique ou une combinaison de ces radicaux ayant de 6 à 30 atomes de carbone, un ou plusieurs atomes de carbone pouvant être remplacés par O, O(C=O), (C=O), Si, N, S, et un groupe époxy cycloaliphatique étant contenu,
Z, Z' représentent, indépendamment l'un de l'autre, H ou un radical aliphatique ramifié ou non ramifié, cycloaliphatique ou aromatique ou une combinaison de ces radicaux ayant de 0 à 10 atomes de carbone, un ou plusieurs atomes de carbone pouvant être remplacés par O, O(C=O), (C=O), Si, N, S,
D représente un radical aliphatique ramifié ou non ramifié, cycloaliphatique ou aromatique ou une combinaison de ces radicaux ayant de 1 à 55 atomes de carbone, au moins un atome de carbone est remplacé par SiGG', SiG ou Si et un ou plusieurs atomes de carbone pouvant être remplacés par O, O(C=O), (C=O), N ou S,
G, G' représentent, indépendamment l'un de l'autre, un radical aliphatique ramifié ou non ramifié, cycloaliphatique ou aromatique ou une combinaison de ces radicaux ayant de 1 à 15 atomes de carbone, un ou plusieurs atomes de carbone pouvant être remplacés par O, O(C=O), (C=O), Si, N, S,
n et m représentent, indépendamment l'un de l'autre, 1, 2, 3 ou 4,
la masse moléculaire de l'époxyde ou la masse moléculaire moyenne du mélange d'époxydes allant de 360 à 3 000 g/mole,
(b) 0 à 80 % en poids d'un époxyde ou d'un mélange d'époxydes, qui sont différents de (a),
(c) 3 à 85 % en poids de charges,
(d) 0,01 à 25 % en poids d'amorceurs, de ralentisseurs et/ou d'accélérateurs,
(e) 0 à 25 % en poids d'adjuvants,
les pourcentages étant chacun par rapport au poids total de la préparation.

2. Préparation polymérisable selon la revendication 1, contenant :
(a) 3 à 50 % en poids d'un époxyde ou d'un mélange d'époxydes de formule générale : 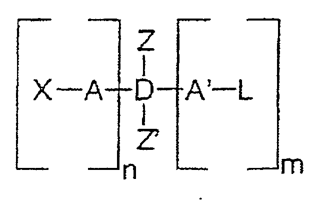 dans laquelle
A, A' représentent, indépendamment l'un de l'autre, un radical aliphatique ramifié ou non ramifié, cycloaliphatique ou aromatique ou une combinaison de ces radicaux ayant de 0 à 12 atomes de carbone, un ou plusieurs atomes de carbone pouvant être remplacés par O, O(C=O), (C=O), Si, N, S,
X représente un radical aliphatique ramifié ou non ramifié ou aromatique ou une combinaison de ces radicaux ayant de 3 à 14 atomes de carbone, un ou plusieurs atomes de carbone pouvant être remplacés par O, O(C=O), (C=O), Si, N, S, et un groupe époxy aliphatique en milieu ou en bout de chaîne étant contenu,
L représente un radical aliphatique ramifié ou non ramifié, cycloaliphatique ou aromatique ou une combinaison de ces radicaux ayant de 6 à 20 atomes de carbone, un ou plusieurs atomes de carbone pouvant être remplacés par 0, O(C=O), (C=O), Si, N, S, et un groupe époxy cycloaliphatique étant contenu,
Z, Z' représentent, indépendamment l'un de l'autre, H ou un radical aliphatique ramifié ou non ramifié, cycloaliphatique ou aromatique ou une combinaison de ces radicaux ayant de 0 à 10 atomes de carbone, un ou plusieurs atomes de carbone pouvant être remplacés par O, O(C=O), (C=O), Si,
D représente un radical aliphatique ramifié ou non ramifié, cycloaliphatique ou aromatique ou une combinaison de ces radicaux ayant de 1 à 50 atomes de carbone, au moins un atome de carbone est remplacé par SiGG', SiG ou Si et un ou plusieurs atomes de carbone pouvant être remplacés par O, O(C=O), (C=O),
G, G' représentent, indépendamment l'un de l'autre, un radical aliphatique ramifié ou non ramifié, cycloaliphatique ou aromatique ou une combinaison de ces radicaux ayant de 1 à 8 atomes de carbone, un ou plusieurs atomes de carbone pouvant être remplacés par O, O(C=O), (C=O), Si,
n et m représentent, indépendamment l'un de l'autre, 1, 2, 3 ou 4,
la masse moléculaire de l'époxyde ou la masse moléculaire moyenne du mélange d'époxydes allant de 360 à 2 500 g/mole,
(b) 0 à 60 % en poids d'un époxyde ou d'un mélange d'époxydes, qui sont différents de (a),
(c) 15 à 85 % en poids de charges,
(d) 0,01 à 20 % en poids d'amorceurs, de ralentisseurs et/ou d'accélérateurs,
(e) 0 à 25 % en poids d'adjuvants,
les pourcentages étant chacun par rapport au poids total de la préparation.

3. Préparation polymérisable selon la revendication 1 ou 2, contenant, en tant que composant (a) un ou plusieurs des époxydes suivants : 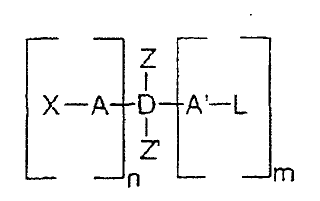 où
A, A' représentent, indépendamment l'un de l'autre, un radical aliphatique ramifié ou non ramifié, cycloaliphatique ou aromatique ou une combinaison de ces radicaux ayant de 0 à 12 atomes de carbone, un ou plusieurs atomes de carbone pouvant être remplacés par O, O(C=O), (C=O), Si, N, S,
X représente un radical aliphatique ramifié ou non ramifié ou une combinaison de ces radicaux ayant de 3 à 14 atomes de carbone, un ou plusieurs atomes de carbone pouvant être remplacés par O, O(C=O), (C=O), Si, N, S, et au moins un groupe époxy aliphatique en bout de chaîne étant contenu,
L représente un radical aliphatique ramifié ou non ramifié, cycloaliphatique ou une combinaison de ces radicaux ayant de 7 à 15 atomes de carbone, un ou plusieurs atomes de carbone pouvant être remplacés par 0, O(C=O), (C=O), Si, N, S, et un groupe époxy cycloaliphatique de formule suivante 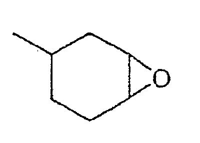 étant contenu,
D représente un radical aliphatique ramifié ou non ramifié, cycloaliphatique ou aromatique ou une combinaison de ces radicaux ayant de 1 à 50 atomes de carbone, au moins un atome de carbone est remplacé par SiGG', SiG ou Si et un ou plusieurs atomes de carbone pouvant être remplacés par O, O(C=O), (C=O),
G, G' représentent, indépendamment l'un de l'autre, un radical aliphatique ramifié ou non ramifié ou aromatique ou une combinaison de ces radicaux ayant de 1 à 8 atomes de carbone, un ou plusieurs atomes de carbone pouvant être remplacés par O, O(C=O), (C=O), Si,
n et m représentent, indépendamment l'un de l'autre, 1, 2, 3 ou 4,
la masse moléculaire de l'époxyde ou la masse moléculaire moyenne du mélange d'époxydes allant de 360 à 2 500 g/mole.

4. Préparation polymérisable selon l'une quelconque des revendications 1 à 3, contenant en tant que composant (a) un ou plusieurs des époxydes suivants : 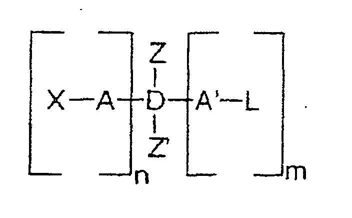 où
A, A' représentent, indépendamment l'un de l'autre, un radical aliphatique ramifié ou non ramifié, cycloaliphatique ou aromatique ou une combinaison de ces radicaux ayant de 0 à 10 atomes de carbone, un ou plusieurs atomes de carbone pouvant être remplacés par O, O(C=O), (C=O), Si,
X représente un radical aliphatique ramifié ou non ramifié ayant de 3 à 14 atomes de carbone, dans lequel un ou plusieurs atomes de carbone peuvent être remplacés par 0, O(C=O), (C=O), Si, et au moins un groupe époxy aliphatique en bout de chaîne est contenu,
L représente un radical aliphatique ramifié ou non ramifié ou cycloaliphatique ou une combinaison de ces radicaux ayant de 7 à 15 atomes de carbone, dans lequel un ou plusieurs atomes de carbone peuvent être remplacés par O, O(C=O), (C=O), Si, et un groupe époxy cycloaliphatique de formule suivante 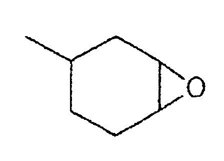 est contenu,
D représente un radical aliphatique ramifié ou non ramifié, cycloaliphatique ou aromatique ou une combinaison de ces radicaux ayant de 1 à 50 atomes de carbone, au moins un atome de carbone est remplacé par SiGG', SiG ou Si et un ou plusieurs atomes de carbone pouvant être remplacés par O, O(C=O), (C=O),
G, G' représentent, indépendamment l'un de l'autre, un radical aliphatique ramifié ou non ramifié ou aromatique ou une combinaison de ces radicaux ayant de 1 à 8 atomes de carbone,
n et m représentent, indépendamment l'un de l'autre, 1, 2, 3 ou 4, et
la somme m + n a une valeur de 2 à 6,
la masse moléculaire de l'époxyde ou la masse moléculaire moyenne du mélange d'époxydes allant de 360 à 2 500 g/mole.

5. Préparation polymérisable selon l'une quelconque des revendications 1 à 4, dans laquelle le composant (a) comporte l'un des constituants D suivants qui est lié aux constituants A et/ou A' par l'atome de silicium
i. Si
ii. SiG
iii. SiGG' 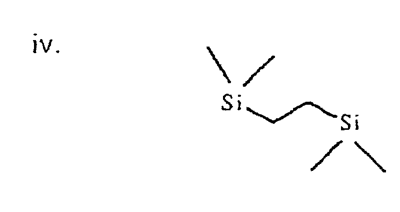 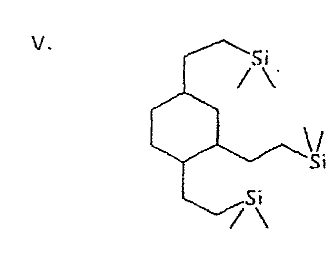 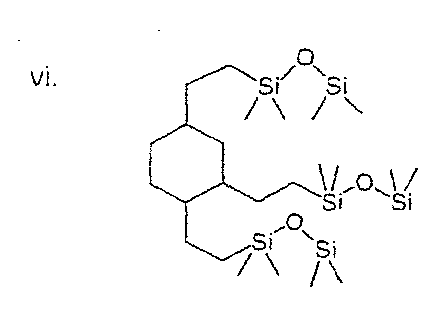 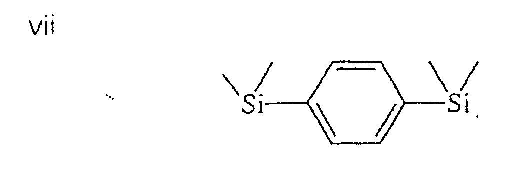 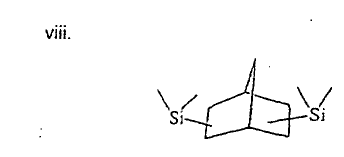 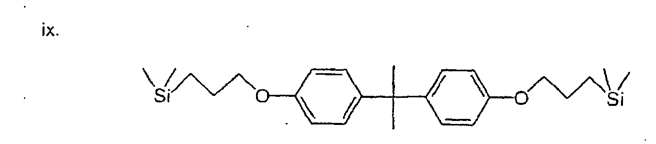 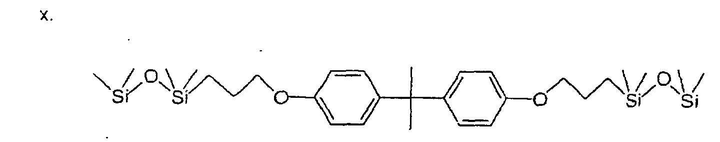 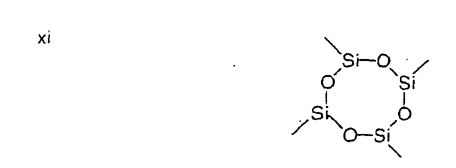 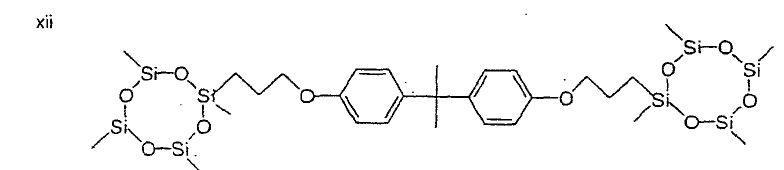 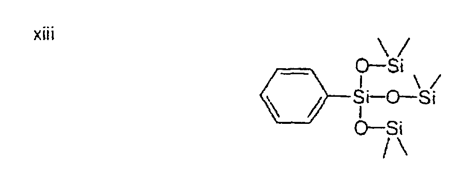
G, G' représentant, indépendamment l'un de l'autre, un radical aliphatique ramifié ou non ramifié, cycloaliphatique ou aromatique ou une combinaison de ces radicaux ayant de 1 à 8 atomes de carbone, un ou plusieurs atomes de carbone pouvant être remplacés par O, O(C=O), (C=O), Si.

6. Préparation polymérisable selon l'une quelconque des revendications 1 à 5, contenant, en tant que composant (a), un ou plusieurs des époxydes suivants :
i. 1,2-éthylène-[1,2-diméthyl-1-[3-(oxirannylméthoxy)propyl]-2-[2-(7-oxabicyclo[4.1.0]hept-3-yl)éthyl]silane 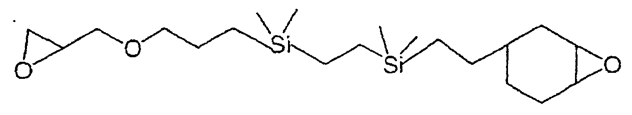
ii. 1,1',1"-(1,2,4-cyclohexylène-1-(diméthyl-[3-(oxirannylméthoxy)propyl])-2,4-bis[2-(7-oxabicyclo[4.1.0]hept-3-yl)éthyl])silane 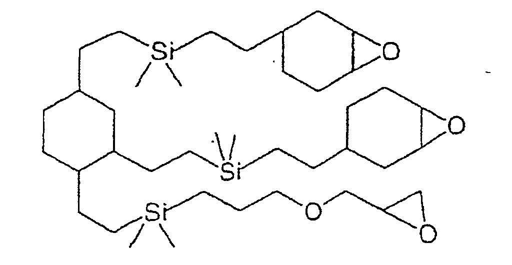
iii. 1,1',1"-(1,2,4-cyclohexanetriyltri-2,1-éthane-diyl)-1,1,3,3-tétraméthyl-3-(oxirannylméthoxy)-propyl-bis[1,1,3,3-tétraméthyl-2-(7-oxabicyclo-[4.1.0]hept-3-yl)éthyl]disiloxane 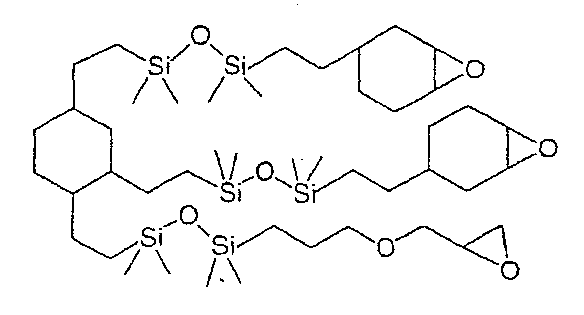
iv. 1,4-phénylène-bis[diméthyl-1-[3-(oxirannylméthoxy)propyl-4-[2-(7-oxabicyclo[4.1.0]hept-3-yl)éthyl]silane 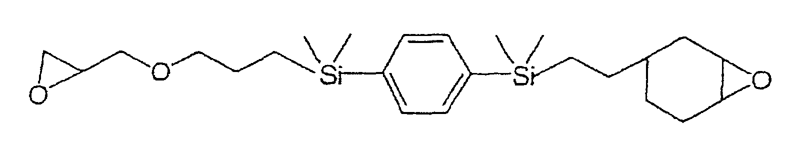
v. 2,5-bicyclo[2.2.1]heptylène-bis[diméthyl-2-[3-(oxirannylméthoxy)propyl]-5-[2-(7-oxabicyclo[4.1.0]hept-3-yl)éthyl]silane 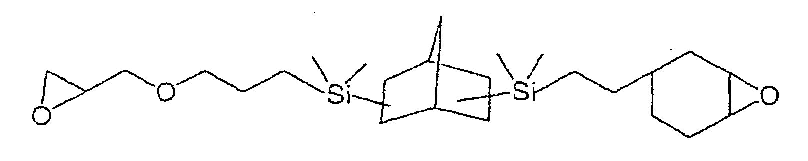
vi. 1,1'-[bis(4,4'-hydroxyphényl-2,2-propane-3,1-propanediyl)]-1-diméthyl-[3-(oxirannylméthoxy)-propyl]-1'-diméthyl-[2-(7-oxabicyclo[4.1.0]hept-3-yl)éthyl]silane 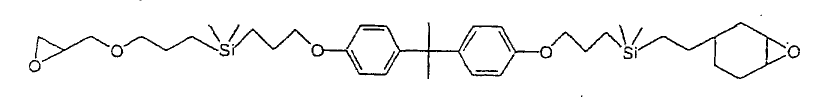
vii. 1,1'-[bis(4,4'-hydroxyphényl-2,2-propane-3,1-propanediyly)-1,1,3,3-tétraméthyl)-1-[3-(oxirannylbutyl)]-1'-[2-(7-oxabicyclo[4.1.0]hept-3-yl)éthyl]siloxane 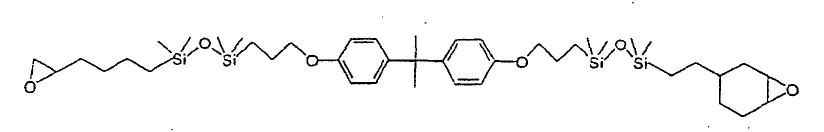
viii. 2,4,6,8-tétraméthyl-2,4,6-tris[2-(7-oxabicyclo[4.1.0]hept-3-yl)éthyl)-8-[3-(oxirannylméthoxy)-propyl]cyclotétrasiloxane 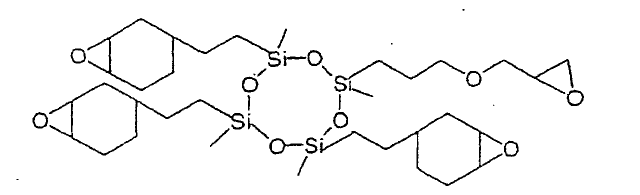
ix. 2,4,6,8-tétraméthyl-2,4,6-tris[2-(7-oxabicyclo[4.1.0]hept-3-yl)éthyl]-8-[4-oxirannylbutyl]-cyclotétrasiloxane 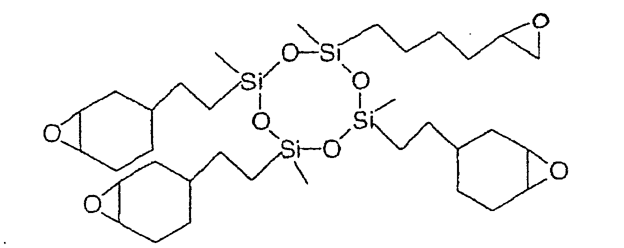
x. 1,1'-[bis(4,4'-hydroxyphényl-2,2-propane-3,1-propanediyl)]-bis[2,4,6,8-tétraméthyl-4,6-bis[3-(oxirannylméthoxy)propyl]-8-[2-(7-oxabicyclo[4.1.0]hept-3-yl)éthyl]cyclotétrasiloxane 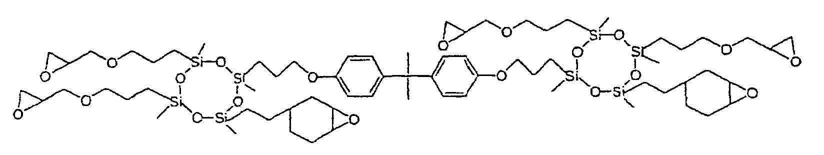
xi. 3-[[diméthyl-[2-(7-oxabicyclo[4.1.0]hept-3-yl)-éthyl]silyl]oxy]-1,1,5,5-tétraméthyl-1-[2-(7-oxabicyclo[4.1.0]hept-3-yl)éthyl]-5-(oxirannylméthoxy)propyl]-3-phényl-trisiloxane 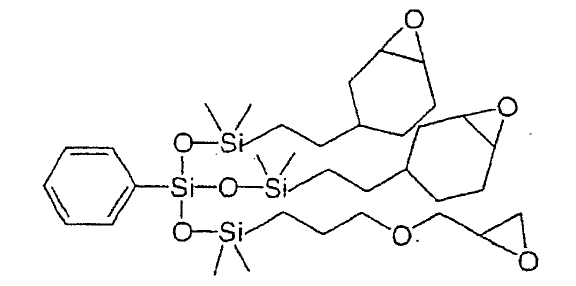
xii. 3-[[diméthyl-[2-(7-oxabicyclo[4.1.0]hept-3-yl)-éthyl]silyl]oxy]-1,1,5,5-tétraméthyl-1-[2-(7-oxabicyclo[4.1.0]hept-3-yl)éthyl]-5-[2-(oxirannyléthyl)]-3-phényl-trisiloxane

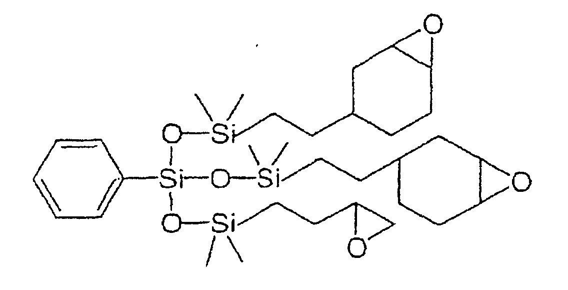

7. Préparation polymérisable selon l'une quelconque des revendications 1 à 6, contenant en tant que charges selon le composant (c) du quartz, des verres broyés, des gels de silice et/ou des acides siliciques, leurs produits granulés et/ou des matières plastiques broyées.

8. Préparation polymérisable selon l'une quelconque des revendications 1 à 7, contenant en tant qu'amorceurs des acides de Lewis et/ou des acides de Brönsted ou des composés à partir desquels sont formés de tels acides par irradiation avec de la lumière UV, de la lumière visible, sous pression et/ou à chaud, ou par réaction chimique.

9. Préparation polymérisable selon l'une quelconque des revendications 1 à 8, contenant en tant qu'accélérateurs des époxydes à fonction hydroxy.

10. Préparation polymérisable selon l'une quelconque des revendications 1 à 9, contenant en tant qu'adjuvants des diluants, des stabilisants, des inhibiteurs et/ou des pigments.

11. Préparation polymérisable selon l'une quelconque des revendications 1 à 10, contenant
A au moins une pâte de base contenant des époxydes ou des mélanges d'époxydes des composants (a) et (b), une partie ou la quantité totale des charges du composant (c), éventuellement des retardateurs et/ou des accélérateurs selon le composant (d) et éventuellement des adjuvants du composant (e), ainsi que, séparément dans l'espace de celle-ci,
B au moins une pâte de catalyseur contenant au moins un amorceur selon le composant (d), éventuellement une partie des charges du composant (c) et éventuellement des adjuvants selon le composant (e),
pâte de base et pâte de catalyseur étant mises en réaction l'une avec l'autre pour l'obtention de la préparation polymérisable.

12. Utilisation de la préparation polymérisable selon l'une quelconque des revendications 1 à 11, en tant que composition de revêtement et/ou pour le collage de supports.

13. Utilisation de la préparation polymérisable selon l'une quelconque des revendications 1 à 11, en tant que matérial dentaire.

14. Récipient, en particulier cartouche ou capsule de mélange, contenant une préparation polymérisable selon l'une quelconque des revendications 1 à 11.

15. Dispositif d'application, contenant une préparation polymérisable selon l'une quelconque des revendications 1 à 11.
